# EUROPEAN PATENT APPLICATION

(11) **EP 1 278 065 A1**
(43) Date of publication of application: **22.01.2003**
(21) Application number: 01923972.2
(22) Date of filing: 24.04.2001
(51) Int. Cl.: G01N 33/569

(54) **METHOD OF DETECTING STREPTOCOCCUS SOBRINUS AND ANTIBODY THEREFOR**

(30) Priority: 25.04.2000 JP 2000124070
(71) Applicant: Tokuyama Dental Corporation, Tokyo 110-0016 (JP); School Juridical Person Nihon University, Tokyo 102-8275 (JP)
(72) Inventor: HIRATA, Kouichirou, Tsukuba-shi, Ibaraki 305-0047 (JP); UKAJI, Fumio, Tsukuba-shi, Ibaraki 305-0035 (JP); MATSUSHIGE, Koji, Tsukuba-shi, Ibaraki 305-0051 (JP); HANYU, Naohiro, Tsukuba-shi, Ibaraki 305-0051 (JP); FUKUSHIMA, Kazuo, Nagareyama-shi, Chiba 270-0157 (JP)
(74) Representative: Albrecht, Thomas, Dr.
(86) International application number: JP0103502
(87) International publication number: WO01081927

(57) **Abstract**

The present invention provides an immunological method for the assay of *Streptococcus sobrinus* participating in dental caries, an antibody for use in the method, and a diagnostic kit. As the antibody, there is used an antibody whose binding ability for *Streptococcus sobrinus* is not less than 100 times that for *Streptococcus mutans.*

## Description

### Technical Field

This invention relates to an immunological method for the assay of *Streptococcus sobrinus* (*Streptococcus* may hereinafter be abbreviated as *S.*), a method for diagnosing the degree of risk of dental caries on the basis of the assay, and an antibody and means for use in these methods.

### Background Art

It is generally known that a class of lactic acid fermentative bacteria called mutans streptococci are closely associated with the onset of dental caries. Accordingly, the degree of risk of dental caries is being judged by examining the quantity of mutans streptococci present in the oral cavity of human subjects, and assay kits therefor are commercially available. It is said that there is a risk of dental caries when the concentration of mutans streptococci in saliva is in the range of 10⁵ to 10⁶ cells/ml and there is a high risk when it is greater than 10⁶ cells/ml.

These mutans streptococci are classified into seven types which are serologically and genetically different from each other. Specifically, they are *Streptococcus cricetus* (serotype a), *Streptococcus rattus* (serotype b), *Streptococcus mutans* (serotypes c, e and f). *Streptococcus ferus* (serotype c), *Streptococcus macacae* (serotype c), *Streptococcus sobrinus* (serotypes d and g), and *Streptococcus downey* (serotype h).

Now, it has been proved that, among these *mutans* streptococci, only two bacteria, namely *Streptococcus mutans* and *Streptococcus sobrinus*, exist predominantly in the oral cavity of human beings. Consequently, these two bacteria have attracted attention as mutans streptococci forming cariogenic plaque in human beings, and comparative investigations on the effect of these bacteria on dental caries are being carried forward. As a result, it has been clarified that (i) *Streptococcus mutans* is very frequently isolated from human oral cavities, whereas *Streptococcus sobrinus* is isolated from about 10 to 30% of human oral cavities, i.e., the rates of occurrence of these bacteria in oral cavities are significantly different [Shigeyuki Hamada, Medical Bacteriology (in Japanese) 4:271-314, 1984]; (ii) *Streptococcus mutans* induces dental caries in the pits and crevices of the tooth surface, whereas *Streptococcus sobrinus* induces dental caries not only in the pits and crevices of the tooth surface, but also on a smooth tooth surface (Madison, K.M., J. Dent. Res., 70:38-43, 1991; Hirose, H., Caries Res. 27:292-297, 1993); (iii) *Streptococcus sobrinus* has higher adsorbability to the tooth surface than *Streptococcus mutans* (van der Mei, HC., et al., Caries Res. 25:415-423, 1991); and (iv) *Streptococcus sobrinus* has a greater acid-producing ability than *Streptococcus mutans* (de Soet, J.J., et al., Caries Res. 25:116-122, 1991).

Moreover, it has been reported that, as a result of epidemiological investigations on the correlation between the distribution of *Streptococcus sobrinus* and *Streptococcus mutans* and the incidence of dental caries, human subjects having both *Streptococcus sobrinus* and *Streptococcus mutans* were found to show a significantly higher incidence of dental caries than human subjects having *Streptococcus mutans* alone (Okada, T., et al., J. Dent. Res. 74:501, 1995; Kohler, B., et al., Community Dent. Oral Epidemiol. 15:332-335, 1987; Hirose, H., et al., Caries Res. 27:292-297, 1993).

All of the above-described reports suggest that the ability of *Streptococcus sobrinus* to induce dental caries is greater than that of *Streptococcus mutans.* For this reason, it is considered that, in order to judge the degree of risk of dental caries with higher reliability, it is important to know not only the concentration of mutans streptococci as a whole, but also the presence or absence, and quantity, of *Streptococcus sobrinus* in the oral cavity of human subjects. However, conventional diagnoses of the degree of risk of dental caries have been based on the concentration of mutans streptococci as a whole, and there has been no report that the concentration of *Streptococcus sobrinus* was determined in order to use it as an index to the degree of risk of dental caries.

As to the assay of *Streptococcus sobrinus* itself, there are several reports in which *Streptococcus sobrinus* was assayed according to immunoassay methods using a monoclonal or polyclonal antibody binding specifically with *Streptococcus sobrinus* as shown in the following paragraphs ① to ⑤. These methods include ones in which *Streptococcus sobrinus* is assayed after being isolated according to troublesome processes such as cultivation and colony isolation (paragraphs ① to ③). Where test fluids are directly used without passing through a cultivation process, they include a method which must employ a special detection technique requiring a troublesome operation in order to achieve highly sensitive assays (paragraph ④), and a method by which highly sensitive assays cannot be achieved (paragraph ⑤). Thus, these methods cannot be said to be suitable for general-purpose assay methods for diagnosing the degree of risk of dental caries.
① A method for assaying *Streptococcus sobrinus* independently by forming colonies according to a cultivation process using Mitis-Salivarius medium containing bacitracin (MSB medium), and identifying *Streptococcus sobrinus* colonies according to a biochemical technique (e.g., a sugar fermentation test), an immunological technique using a serotype-specific antibody, or the like (Okada, T., et al., J. Dent. Res. 74:501, 1995; Kohler, B., et al., Community Dent. Oral Epidemiol. 15:332-335, 1987; Hirose, H., et al., Caries Res. 27:292-297, 1993). This method is most commonly employed in epidemiological investigations for examining the relationship between *Streptococcus sobrinus* and dental caries.
② A method for assaying various reference strains and clinical isolates (single strains isolated from saliva or dental plaque by cultivation) by using a polyclonal antibody specific for *Streptococcus sobrinus* whose binding selectivity for *Streptococcus sobrinus* has been enhanced by an absorption treatment with other mutans streptococci (see, for example, Ota, F., et al., ZbL Bakt. Hyg. A265:330-339, 1987; and Bratthall, D., Odont. Revy. 23:181-196, 1972). The absorption treatment of a polyclonal antibody with other bacteria is a means for enhancing its binding selectivity for the desired antigen (also referred to as its reactivity ratio; the ratio of its binding ability for the desired antigen to its binding ability for other antigens). In the aforesaid method of Ota, F., et al., polyclonal antibodies specific for the serotype d and g strains of *Streptococcus sobrinus* are prepared. Then, the ratio of their binding ability for *Streptococcus sobrinus* to their binding ability for *Streptococcus mutans* is enhanced by an absorption treatment with *Streptococcus mutans*. However, with both polyclonal antibodies, the aforesaid binding ability ratio is in the range of about 18 to 26 based on the binding ability for the *Streptococcus mutans* strain used for the absorption treatment, and in the range of about 15 to 19 based on the average binding ability for all *Streptococcus mutans* strains. In the aforesaid method of Bratthall, a polyclonal antibody is prepared by immunizing an animal with a serotype d reference strain of *Streptococcus sobrinus*, and then subjected to an absorption treatment with a bacterium of serotype a (i.e., *Streptococcus cricetus*). However, no particular investigation is made as to its binding abilities for *Streptococcus* *sobrinus* and other mutans streptococci.
③ A method in which *Streptococcus sobrinus* present in saliva or dental plaque is once cultured to raise its cell concentration, and then assayed with the aid of a monoclonal antibody against *Streptococcus sobrinus* (de Soet, J.J., J. Clin. Microbiol. 28:2467-2472, 1990). In this reference, it is described that, since the aforesaid monoclonal antibody has a low binding ability, it is impossible to detect *Streptococcus sobrinus* present in dental plaque directly by a fluorescent antibody technique.
④ A method for assaying *Streptococcus sobrinus by* a fluorescent antibody technique using a polyclonal antibody (Walter, J., J. Dent. Res. 55:A87-A93, 1976; Babaahmady, K.G., Caries Res. 32:51-58, 1998). This method does not require a cultivation process, but requires a troublesome technique in which an antibody having bound with *Streptococcus mutans* and an antibody having bound with *Streptococcus sobrinus* must be distinguished on the basis of fluorescence intensity by visual inspection using a special apparatus such as a fluorescence microscope.
⑤ A method for assaying *Streptococcus sobrinus* present in saliva or dental plaque directly by a latex agglutination technique using a polyclonal antibody against *Streptococcus sobrinus* (Tsutomu Takei, Handai Igaku Zasshi (Osaka University Journal of Medicine) 35:93-109, 1990; Takei, T., Archs. oral. Biol. 37:99-104, 1992; Japanese Patent Laid-Open No. 250067/'89). The lower detection limit in this method is not less than 1x10⁶ cells/ml and, therefore, its measuring sensitivity is insufficient. Generally, when an antigenic bacterium present is assayed by using a polyclonal antibody prepared by immunizing an animal with the antigenic bacterium, the antibody usually has a high binding ability to the bacterium because of the strong antigenicity of the bacterium. Consequently, it is considered relatively easy to detect bacteria contained in test fluids, for example, at a concentration of the order of 10³ cells/ml. For example, there have been known many latex agglutination tests in which 10⁵ cells/ml of bacteria were successfully detected by using a polyclonal antibody similar to that used in the above method, but the above method for the assay of *Streptococcus sobrinus* failed to achieve sufficient sensitivity. The reason for this is considered to be that, since other bacteria were not removed by such processes as cultivation and colony isolation, the polyclonal antibody used therein showed a cross reaction with other mutans streptococci and, therefore, a sufficient amount of antibody could not be applied to the latex agglutination test (if the amount of antibody used is increased, a reaction with *Streptococcus mutans* will be detected).

For a general-purpose assay method for diagnosing the degree of risk of dental caries, it is considered important that clinical samples prepared directly from saliva or dental plaque can be used as they are, and a highly sensitive assay which permits the detection of *Streptococcus sobrinus* in a concentration, for example, of the order of 10⁵ cells/ml can be carried out according to a simple procedure. In the existing state of the art, however, an assay method for *Streptococcus sobrinus* which meets these requirements is not yet known as described above.

Accordingly, an object of the present invention is to provide a method by which *Streptococcus sobrinus* present in test fluids prepared directly from saliva or dental plaque collected from the oral cavity and suspected of containing *Streptococcus mutans* and *Streptococcus sobrinus* can be rapidly and simply assayed with high sensitivity and without requiring a troublesome process such as a cultivation process.

### Disclosure of the Invention

Considering that one of the causes for a failure to enhance detection sensitivity is the influence of contaminants (e.g., oral streptococci) present in test fluids, the present inventors focused their attention on *Streptococcus mutans* among a variety of oral streptococci and made investigations on its influence. Specifically, antibodies having different ratios of reactivity with *Streptococcus sobrinus* to reactivity with *Streptococcus mutans* were prepared and used to make various investigations on the ratio of reactivity and the detection sensitivity.

As a result of these investigations, it has been found that, when a highly sensitive antibody whose binding ability for *Streptococcus sobrinus* is not less than 100 times that for *Streptococcus mutans*, which was obtained for the first time by the present inventors, is used, *Streptococcus sobrinus* can be detected and assayed even if the concentration of *Streptococcus sobrinus* in test fluids is of the order of 10⁵ cells/ml. The present invention has been completed on the basis of this finding.

Thus, according to the present invention, there is provided a method for the detection of *Streptococcus sobrinus* in a test fluid suspected of containing *Streptococcus sobrinus* and *Streptococcus mutans,* said method comprising the steps of
(A) providing an antibody whose binding ability for *Streptococcus sobrinus* is not less than 100 times that for *Streptococcus mutans*;
(B) bringing the antibody into contact with the test fluid to form an immune complex; and
(C) assaying the immune complex.

According to another embodiment of the present invention, there is provided a diagnostic method for judging the degree of risk of dental caries in a human subject, said method comprising the steps of
(a) preparing a test fluid derived from the saliva and/or dental plaque of the subject;
(b) providing an antibody whose binding ability for *Streptococcus* *sobrinus* is not less than 100 times that for *Streptococcus mutans*;
(c) bringing the test fluid prepared in step (a) into contact with the antibody provided in step (b) to form an immune complex; and
(d) assaying the immune complex, and evaluating its amount as an index to the risk of dental caries.

According to still another embodiment of the present invention, there is provided a diagnostic method as described above wherein step (c) is carried out in the coexistence of the antibody (S antibody) with an antibody combining specifically with *Streptococcus mutans* (M antibody), or in addition to step (c), another step similar to step (c) is carried out by using M antibody in place of S antibody; the resulting immune complex derived from M antibody is also assayed; and the amount of this complex is also evaluated as an index to the risk of dental caries.

According to a further embodiment of the present invention, there is provided an immunoassay kit or a diagnostic kit for judging the degree of risk of dental caries in human subjects, said kit including an antibody whose binding ability for *Streptococcus sobrinus* is not less than 100 times that for *Streptococcus mutans*; and if necessary, an antibody combining specifically with *Streptococcus mutans*, or an antibody combining specifically with *Streptococcus mutans* and *Streptococcus sobrinus* (MS antibody).

According to still a further embodiment of the present invention, there is provided an immunochromatographic strip comprising a sample pad for absorbing and holding a test fluid temporarily, a conjugate pad for holding a labeled antibody temporarily, and a development membrane having a detection antibody immobilized thereto and allowing the development of the test fluid absorbed and held temporarily in the sample pad and the labeled antibody flowing out of the conjugate pad together with the test fluid, wherein the sample pad, the conjugate pad and the development membrane are joined together in the order mentioned, said immunochromatographic strip being characterized in that an antibody whose binding ability for *Streptococcus sobrinus* is not less than 100 times that for *Streptococcus mutans* is used as the detection antibody.

According to still a further embodiment of the present invention, there is provided a polyclonal antibody whose binding ability for *Streptococcus sobrinus* is not less than 100 times that for *Streptococcus mutans.*

The above-described method of the present invention makes it possible to assay *Streptococcus sobrinus* present in saliva or dental plaque directly by an immunoassay technique without culturing *Streptococcus sobrinus*, as illustrated in the examples which will be given later. This assay has been made possible on the basis of the fact that the properties of an antibody required for a highly sensitive assay have been elucidated by the present inventors and a novel antibody meeting such property requirements has been discovered thereby.

In the method of the present invention, the quantity of *Streptococcus sobrinus* present in test fluids and the quantity of *Streptococcus mutans* present therein can simultaneously be determined by the concurrent use of an antibody combining specifically with *Streptococcus mutans.* Moreover, the quantity of *Streptococcus sobrinus* present in test fluids and the total quantity of *Streptococcus mutans* and *Streptococcus sobrinus* present therein can simultaneously be determined by the concurrent use of an antibody combining specifically with *Streptococcus mutans* and *Streptococcus sobrinus.*

If desired, the above-described kit or strip of the present invention may further include an antibody binding specifically with *Streptococcus mutans,* or an antibody binding specifically with *Streptococcus mutans* and *Streptococcus sobrinus*. Thus, this kit or strip makes it possible to determine the quantity of *Streptococcus sobrinus*, and the quantity of *Streptococcus mutans* or the total quantity of *Streptococcus mutans* and *Streptococcus sobrinus* at the same time.

### Brief Description of the Drawings

FIG. 1 is a schematic view of several components of a strip for use in the immunochromatographic technique of the present invention. In this figure, reference numeral 2 designates a sample pad; 3, a conjugate pad; 4, a development membrane; 5, an absorption pad; 6, a detection line; and 7, a control judgment line.
FIG. 2 is a side view of the strip shown in FIG. 1.
FIGs. 3 and 4 are schematic views of strips for use in the immunochromatographic technique of the present invention in which M antibody or MS antibody is concurrently used. The reference numerals in this figure correspond to those in FIG. 1.

### Best Mode for Carrying Out the Invention

The method of the present invention makes it possible to assay *Streptococcus sobrinus* contained in test fluids suspected of containing *Streptococcus sobrinus* and *Streptococcus mutans*. Although these test fluids may be any of natural and artificial ones, they usually comprise liquid samples prepared from saliva or dental plaque by dissolving, diluting or concentrating it as required.

As used herein, the term *"Streptococcus sobrinus*" means one or both types of mutans streptococci which are classified into serotypes d and g on the basis of the structure of the cell surface polysaccharide antigen. Examples of the reference strains of serotype d include B13 and OMZ176 strains, and examples of the reference strains of serotype g include 6715 and OMZ65 strains.

As used herein, the term "*Streptococcus mutans*" means those types of mutans streptococci which are classified into serotypes c, e and f. Examples of the reference strains of serotype c include Ingbritt and MT6R strains, examples of the reference strains of serotype e include LM7 and P4 strains, and examples of the reference strains of serotype f include SE11 and OMZ175 strains.

The reference strains described herein are ones which have long been recognized widely as common reference strains and are being extensively used for various purposes (see, for example, Ono, T., et al., J. Med. Microbiol. 41:231-235, 1994; Perch, B., Acta path. microbiol. scand. 82:357-370, 1974; and Bratthall, D., Odont. Revy. 21:143-152, 1970). Specifically, for *Streptococcus mutans*, it is preferable to use Ingbritt as a reference strain of serotype c, P4 as a reference strain of serotype e, and OMZ175 as a reference strain of serotype f. For *Streptococcus sobrinus*, it is preferable to use B13 as a reference strain of serotype d, and 6715 as a reference strain of serotype g. These reference strains can be relatively easily obtained, for example, from the public agencies and the like which are described in the aforementioned article by Ota, F., et al.

According to the detection method of the present invention, *Streptococcus sobrinus* can be detected for test fluids containing *Streptococcus sobrinus* at a concentration of not less than 10⁵ cells/ml. The concentration (in cells/ml) of *Streptococcus sobrinus* in test fluids can be determined by counting *Streptococcus sobrinus* according to a conventionally known bacteriological technique such as a cultivation process. For example, in order to determine its concentration by a cultivation process, a suspension obtained by diluting a test fluid suitably is subjected to a cell dispersion treatment (e.g., ultrasonication) and then spread on a plate of Brain Heart Infusion medium (which may hereinafter be referred to as "BHI medium"). Thus, the concentration (in cells/ml) of *Streptococcus* *sobrinus* in the test fluid can be determined by counting the number of colonies of *Streptococcus sobrinus* so formed is counted and multiplying it by the dilution ratio of the test fluid. When the test fluid contains *Streptococcus mutans* and other oral bacteria in addition to *Streptococcus sobrinus*, it is necessary to classify the colonies formed on the medium plate accurately into colonies of *Streptococcus sobrinus* and colonies of other bacteria, and count only the colonies of *Streptococcus sobrinus*. The classification of colonies can be carried out by applying a biochemical technique (e.g., a sugar fermentation test), an immunological technique using a specific antibody, or a genetic technique using a DNA probe to the colonies formed on the medium plate.

No particular limitation is placed on the concentration of *Streptococcus mutans* in test fluids for use in the method of the present invention. However, from the viewpoint of detection sensitivity for *Streptococcus sobrinus*, it is preferably in the range of 0 to 10⁸ cells/ ml and more preferably 10⁵ to 10⁷ cells/ml. Its concentration can be determined in the same manner for the concentration of *Streptococcus sobrinus.*

As the test fluids that is the fluid samples should be tested in the method of the present invention, particularly for the purpose of diagnosing the degree of risk of dental caries, it is especially preferable to use test samples containing saliva or dental plaque. Moreover, from the viewpoint of the ease of preparation of test fluids, it is especially preferable to use test samples prepared directly from saliva or dental plaque without passing through a cultivation process. In the test samples, saliva and dental plaque may be contained alone or in admixture.

For example, a test fluid containing dental plaque alone may be prepared from dental plaque collected after the oral cavity is washed (e.g., by gargling) to remove saliva components therefrom. Dental plaque may be collected from selected sites in the oral cavity with a conventionally known means such as a toothpick, swab or spatula, or may be collected from the oral cavity at random. The dental plaque collected in this manner may be suspended in a liquid to prepare a test fluid. As the liquid, any aqueous liquid may be used without restriction, and usable aqueous liquids include, for example, purified water, physiological saline and various conventionally known buffer solutions. However, it is especially preferable to use a buffer solution so that an antigen-antibody reaction may be effected under constant optimum conditions. As the buffer solution, conventionally known buffer solutions having a buffer capacity in the pH range of 6.0 to 8.0 may be used without any restriction, and usable buffer solutions include, for example, phosphate buffer solutions, Tris buffer solutions, Good's buffer solutions (e.g., HEPES) and borate buffer solutions. As to the amount of the liquid, dental plaque can be uniformly suspended by using the liquid in an amount of 0.1 to 10 ml per mg of dental plaque. Generally, in cases which are diagnosed with the result that there is "a risk of dental caries", the concentrations of *Streptococcus sobrinus* in dental plaque and saliva are 10⁵ cells per mg of wet dental plaque and 10⁵ cells per ml of saliva, respectively. Consequently, with consideration for the cases in which the degree of risk of dental caries is diagnosed by using saliva directly as a test fluid, it is most preferable to use 1 ml of the liquid per mg of dental plaque. Although a test fluid containing dental plaque may be directly used, it is preferable to use the test fluid after being subjected to a conventionally known treatment for dispersing the dental plaque, such as ultrasonication or agitation with a grinding medium (e.g., glass beads).

A test fluid containing saliva alone may be prepared from saliva collected by a conventionally known means such as a dropper or pipette. The collected saliva may be directly used as a test fluid, or may be suitably diluted with an aqueous liquid as described above to prepare a test fluid. From the viewpoint of measuring sensitivity, it is preferable to use the collected saliva directly as a test fluid.

Dental plaque or saliva collected from a human subject having a risk of dental caries usually contains *Streptococcus sobrinus* in a quantity of 10⁵ to 10⁷ cells per mg of wet dental plaque or 10⁵ to 10⁷ cells per ml of saliva.

In the method of the present invention, *Streptococcus sobrinus* present in test fluids as described above is assayed according to an immunoassay technique using an antibody characterized in that the ratio of its binding ability for *Streptococcus sobrinus* to its binding ability for *Streptococcus mutans* (i.e., the value obtained by dividing its binding ability for *Streptococcus sobrinus by* its binding ability for *Streptococcus mutans*; hereinafter also referred to as the "S/M binding selectivity") is not less than 100 and in that its binding ability permits its immune complex with *Streptococcus sobrinus* to retain sufficient stability for use in common immunoassays.

The term "antibody" as used herein is not limited to an antibody of a particular globulin class, but comprehends antibodies of any presently known globulin classes. Moreover, it comprehends not only the ordinary antibody molecule, but also partial decomposition products of the antibody (e.g., Fab, Fab' and Fab'2), partial structures containing the active fragment of the antibody (i.e., the antigen-recognition site of the antibody), and the like.

The statement that the S/M binding selectivity is not less than 100 means that, when an antigen-antibody reaction is detected by using *Streptococcus sobrinus or Streptococcus mutans as* the antigen and by employing a conventionally known highly quantitative immunoassay technique such as enzyme immunoassay (which may hereinafter be abbreviated as "ELISA") or radioimmunoassay, the binding ability detected by using *Streptococcus sobrinus* as the antigen is not less than 100 times the binding ability detected by using *Streptococcus mutans* as the antigen. As an index to binding ability, there may be used the measured value (or reaction value) obtained by detecting an immune complex formed by an antigen-antibody reaction according to the aforesaid immunoassay technique, or the amount of antigen (i.e., the quantity of bacterial cells) participating in the antigen-antibody reaction.

In order to determine the S/M binding selectivity of the antibody, an antigen-antibody reaction is effected by using the same quantity of *Streptococcus sobrinus* or *Streptococcus mutans* as the antigen. Then, the reaction value detected when *Streptococcus sobrinus* is used as the antigen is divided by the reaction value detected when *Streptococcus mutans* is used as the antigen. For example, it is assumed that, using 10⁷ cells/ml of *Streptococcus sobrinus* or *Streptococcus mutans* as the antigen, an immune complex is detected by radioimmunoassay. Then, if the reaction value detected by using *Streptococcus sobrinus* as the antigen is 100 and the reaction value detected by using *Streptococcus mutans* as the antigen is 1, the S/M binding selectivity is 100.

Alternatively, when *Streptococcus sobrinus* and *Streptococcus mutans* are used as antigens, the S/M binding selectivity can also be determined by comparing the amounts of antigen (or the quantities of bacterial cells) which give an identical reaction value. For example, it is assumed that, using *Streptococcus sobrinus* or *Streptococcus mutans* as the antigen, an immune complex is detected by ELISA. Then, if the measured value detected by using 10⁶ cells/ml of *Streptococcus sobrinus* as the antigen is 1.0 and the measured value detected by using 10⁸ cells/ml of *Streptococcus mutans* as the antigen is 0.9, the S/M binding selectivity is greater than 100.

The binding ability (also referred to as "reactivity") of the antibody against each bacterial strain can preferably be determined by ELISA using suspensions of reference strains in phosphate-buffered saline (pH 7.4) (which may hereinafter be abbreviated as "PBS") as test fluids. As examples of the reference strains used for this purpose, B13 and 6715 may be used for the serotype d and g strains of *Streptococcus sobrinus*, respectively, and Ingbritt, P4 and OMZ175 may be used for the serotype c, e and f strains of *Streptococcus mutans,* respectively. According to a specific procedure, PBS suspensions of reference strains having a predetermined concentration are separately added to wells of a 96-well immunoplate and adsorbed thereto, followed by blocking. Then, an antibody against *Streptococcus sobrinus* is added to the immunoplate. Thereafter, the binding ability of the antibody against each strain can be determined, for example, by adding an enzyme-labeled secondary antibody and measuring the enzyme activity.

When the binding ability is evaluated by competitive ELISA, PBS solutions of *Streptococcus sobrinus* reference strains having an identical concentration are added to wells of a 96-well immunoplate and adsorbed thereto, followed by blocking. Then, a mixture of an antibody against *Streptococcus sobrinus* and a definite quantity of a reference strain of *Streptococcus sobrinus* or *Streptococcus mutans* is added to the immunoplate. Thereafter, the reactivity of the antibody can be evaluated, for example, by adding a solution of an enzyme-labeled secondary antibody and measuring the enzyme activity.

If an antibody having an S/M binding selectivity of less than 100 is used, a highly sensitive assay cannot be achieved. For example, when an antibody having an S/M binding selectivity of 10 is used, it is assumed that the measured value obtained by using 10⁷ cells/ml of *Streptococcus sobrinus* as the antigen and detecting the immune complex so formed is 100. Then, the measured value obtained by using 10⁶ cells/ml of *Streptococcus sobrinus* as the antigen will be 10. However, the measured value obtained by using 10⁷ cells/ml of *Streptococcus mutans* as the antigen will also be 10. That is, if 10⁷ cells/ml of *Streptococcus mutans* exists even in the absence of *Streptococcus sobrinus*, there is a possibility of judging wrongly that 10⁶ cells/ml of *Streptococcus sobrinus* exists.

Generally, it can be judged that there is "a risk of dental caries" when the concentration of *Streptococcus sobrinus* in saliva is not less than 10⁵ cells/ml. Consequently, a sample showing a *Streptococcus sobrinus* concentration of not less than 10⁵ cells/ml may be defined as "positive for the risk of dental caries". Then, if an antibody having an S/M binding selectivity of less than 100 as described above is used, an unduly large number of samples will be judged to be false positive on the basis of the assay results. For this reason, such an antibody cannot be used in assays for judging the degree of risk of dental caries. The S/M binding selectivity of S antibody is preferably not less than 500 and more preferably not less than 1,000, because with consideration for the possible existence of *Streptococcus mutans* in the oral cavity, the assay result of the corresponding immune complex can be used as an index to the degree of risk of dental caries without producing a high incidence of false positive cases.

No particular limitation is placed on the type of S antibody used in the present invention, so long as its S/M reactivity ratio is not less than 100. However, a polyclonal antibody is preferred from the viewpoint of ease of antibody preparation.

Moreover, in order to detect and determine the total quantity of *Streptococcus sobrinus* present in test fluids, it is preferable that the S antibody have an equal binding ability for the serotype d and g strains of *Streptococcus sobrinus*. As used herein, the statement that the binding abilities for the serotype d and g strains of *Streptococcus sobrinus* is equal to each other means that, when an antigen-antibody reaction is detected according to a conventionally known immunoassay technique by using an equal quantity of the serotype d and g strains of *Streptococcus sobrinus* as antigens, the mutual ratio between the binding abilities obtained from both strains is within 2 and preferably within 1.5.

Furthermore, the S antibody must have a binding ability which permits its immune complex with *Streptococcus sobrinus* to have sufficient stability for ordinary immunoassays. As used herein, the expression "binding ability which permits its immune complex with *Streptococcus sobrinus* to have sufficient stability for ordinary immunoassays" means that, when *Streptococcus sobrinus* is assayed according to a certain immunoassay technique selected from among common immunoassay techniques, the antibody has a binding ability which can detect *Streptococcus sobrinus* in a cell concentration at which an analogous antigen (in this case, *Streptococcus mutans* or other streptococcus, for example) is said to be usually detectable by the same immunoassay technique. For example, when *Streptococcus sobrinus* is assayed according to an immunochromatographic technique using gold colloid as a labeling substance, this means that the antibody has a binding ability which can detect *Streptococcus sobrinus* in a concentration of not less than 10⁵ cells/ml.

This binding ability may also be expressed as the lower detection limit of the cell concentration as determined by direct ELISA using a reference strain of *Streptococcus sobrinus*. It is assumed that, when an assay is made by direct ELISA in which 50 µl each of suspensions of a reference strain of *Streptococcus sobrinus* is used as samples and its cells are immobilized to wells of a 96-well immunoplate and assayed by using 50 µg/well of an antibody, the lower detection limit of the cell concentration is not less than 10⁶ cells/ml (5x10⁴ cells/well) and preferably not less than 2x10⁵ cells/ml (10⁴ cells/well). Then, it may be said that the antibody has a binding ability which permits its immune complex with *Streptococcus sobrinus* to have sufficient stability for ordinary immunoassays.

More specifically, the aforesaid lower detection limit of the cell concentration as determined by direct ELISA can be obtained in the following manner.

6715 (*Streptococcus sobrinus*, serotype g) and B13 (*Streptococcus sobrinus*, serotype d) are suspended in BHI liquid medium and incubated at 37°C under anaerobic conditions. Thereafter, each culture medium is centrifuged and the supernatant comprising medium components is removed to recover the precipitate of bacterial cells. Then, the precipitate of bacterial cells is washed with PBS, resuspended in PBS, ultrasonicated at 40w for 20 seconds, and adjusted to A₆₀₀=1.0 with PBS. Thus, there is obtained a reference strain suspension having a cell concentration of about 1x10⁹ cells/ml. The cell concentration of this reference strain suspension is the value determined by spreading a suitably diluted reference strain suspension over a plate of BHI medium, counting the colonies so formed, and multiplying the resulting count by the dilution ratio. Then, each of the reference strain suspensions (A₆₀₀=1.0) is diluted with a 0.1 M carbonate buffer solution (pH 9.0) at dilution ratios of 1 to 1x10⁵ to prepare a series of tenfold dilutions. 50 µl each of the diluted cell suspensions are added to wells of a 96-well immunoplate and immobilized thereto by allowing the immunoplate to stand at 4°C for 12 hours. After the cell suspensions are removed from the immunoplate, its wells are washed with PBS, followed by blocking. Thereafter, an antibody to be evaluated (i.e., a primary antibody) is diluted with PBS (pH 7.4) containing 1% BSA and 0.05% Tween 20 so as to give an IgG concentration of 1.0 µg/ml, and 50 µl of the antibody solution is added to each well of the immunoplate. After the immunoplate is allowed to stand at 37°C for 1 hour, it is freed of the solution and washed. Thereafter, an alkaline phosphatase-labeled secondary antibody [for example, when the primary antibody is rabbit IgG, alkaline phosphatase-labeled anti-rabbit IgG polyclonal antibody (Cappel; Catalog No. 59652)] is diluted with PBS (pH 7.4) containing 1% BSA and 0.05% Tween 20 so as to give a concentration of 10 µg/ml, and 50 µl of this antibody solution is added to each well of the immunoplate. After the immunoplate is allowed to stand at 37°C for 1 hour, it is freed of the solution and washed. Finally, 50 µl of a color-producing substrate solution, namely an aqueous 2-ethanolamine solution of p-nitrophenyl phosphate [e.g., a color-producing substrate solution manufactured by BIORAD (Catalog No. 172-1063)] is added to each well and reacted at 25°C for 20 minutes. After the reaction is stopped by adding 50 µl of 0.4 M NaOH to each well, the absorbance of each well at 405 nm and the background absorbance are measured, and the concentration of cells immobilized to the well showing an absorbance equal to the background absorbance is regarded as the lower detection limit.

As the S antibody used in the present invention, it is especially preferable to use a monoclonal or polyclonal antibody characterized in that its S/M reactivity ratio is not less than 1,000, the mutual ratio between its binding abilities for the serotype d and g strains of *Streptococcus sobrinus* is within 2 and preferably within 1.5, and its reactivity with *Streptococcus sobrinus* is not less than 10⁶ cells/ml and more preferably 2x10⁵ cells/ml when expressed as the aforesaid lower detection limit of the cell concentration. With consideration for the ease of antibody preparation, it is most preferable to use a polyclonal antibody having such properties.

When the S antibody comprises a polyclonal antibody, it may preferably be prepared by immunizing an immune animal with whole cells of *Streptococcus sobrinus* or an antigenic extract obtained from the whole cells, harvesting the resulting polyclonal antibody against *Streptococcus sobrinus*, and then removing therefrom polyclonal antibodies having cross reactivity with *Streptococcus mutans*.

When the S antibody comprises a monoclonal antibody, it may be prepared by immunizing a mammal with an antigen as described above, separating antibody-producing cells (e.g., spleen cells or lymphocytes) therefrom, fusing the separated antibody-producing cells with myeloma cells to form hybridomas, selecting a hydridoma capable of producing an antibody having an S/M reactivity ratio of not less than 100, and harvesting the culture supernatant of the selected hybridoma. Alternatively, the S antibody may be prepared by transplanting the aforesaid hydridoma to the intraperitoneal cavity of an animal, allowing it to grow, and collecting the resulting ascites. As used herein, the term "monoclonal antibody" also comprehends recombinant antibodies prepared by culturing a recombinant into which all or part of the antibody gene of the hybridoma has been introduced.

Among these antibodies, polyclonal antibodies are easy to prepare. Now, the method for the preparation of S antibody comprising a polyclonal antibody is specifically described below.

In order to prepare S antibody comprising a polyclonal antibody, whole cells of the serotype d or g strain of *Streptococcus sobrinus*, an antigen extracted from these whole cells, or a mixture thereof may be used as an immunizing antigen.

As the whole cells, there may be used not only living cells, but also dead cells having been subjected to formalin treatment or heat treatment, and cryopreserved cells.

When the antigen is a polysaccharide antigen, its extraction from whole cells may be carried out according to any of conventionally known methods such as a method comprising the heat treatment of a cell suspension (Rants, L.A., Stanford Med. Bull. 13:290-291, 1955) and a method comprising extraction with nitrous acid [Tsutomu Takei, Osaka University Journal of Medicine (in Japanese) 35:93-109, 1990]. The extracted polysaccharide antigen may be directly used as an antigen, or may be used after further purification. When the antigen is a protein antigen, it may be obtained by extracting an antigenic protein with an alkali, salt, chelating agent, chaotropic ion or surface-active agent [Tsutomu Takei, Osaka University Journal of Medicine (in Japanese) 35:93-109, 1990] or by purifying a specific protein present among proteins derived from bacterial cells (Okahashi, N., Microbiol. Immunol. 30:35-47, 1986; Hamada, S., J. Gen. Microbiol. 135:335-344, 1989). Moreover, there may also be used recombinant proteins prepared by culturing a recombinant into which all or part of the gene encoding a specific protein derived from bacterial cells has been introduced, synthetic peptides synthesized on the basis of the amino acid sequence of a specific protein, and the like.

These antigenic extracts and the like may be directly used as antigens, or may be used after being combined with an immunizing carrier. As the carrier, there may preferably be used any of conventionally known carriers such as keyhole limpet hemocyanin (KLH), bovine serum albumin (BSA), human serum albumin (HSA), avian serum albumin, poly-L-lysine, polyalanyllysine, dipalmityllysine, tetanus toxoid and polysaccharides.

As the antigen used to prepare a polyclonal antibody used as S antibody in the present invention, it is preferable from the viewpoint of simplicity of operation to use bacterial cells directly or use a polysaccharide antigen extract directly. Moreover, it is especially preferable to use whole cells of *Streptococcus sobrinus* directly for immunizing purposes, because the antibody thus obtained has a high titer.

As the animal immunized with an antigen as described above, there may be used any of various animals commonly used for the preparation of antibodies. However, it is preferable to use mammals such as mice, goats, rabbits and guinea pigs. As the method for immunizing such an animal with an antigen, any of conventionally known methods may be employed without restriction. Specifically, the animal may be immunized directly with bacterial cells or an extract therefrom, or may be immunized with a mixture of such an antigen and an adjuvant. For example, when the animal is immunized directly with bacterial cells, a cell suspension obtained after *Streptococcus sobrinus* is cultured in BHI liquid medium or the like may be used as it is. As the adjuvant, there may preferably be used any of conventionally known adjuvants such as Freund's complete adjuvant, Freund's incomplete adjuvant, aluminum hydroxide adjuvant and pertussis adjuvant.

A fluid containing a polyclonal antibody against *Streptococcus sobrinus (this* fluid may hereinafter be referred to as the "antiserum") can be obtained by immunizing such an animal with an antigen, collecting blood from the animal, and preparing serum therefrom.

The resulting antiserum may be directly used in the immunoassay method, provided that the polyclonal antibody contained in the antiserum has an S/M binding selectivity of not less than 100. However, the antiserum usually contains not only an antibody specific for *Streptococcus sobrinus*, but also other antibodies and proteins other than antibodies (e.g., albumin). Accordingly, in order to achieve a highly sensitive and specific assay, it is preferable to separate a polyclonal antibody fraction by treating the resulting antiserum by a technique such as salting out, gel filtration, ion-exchange chromatography, affinity chromatography or electrophoresis. For example, when a rabbit is immunized with an antigen comprising cells of *Streptococcus sobrinus*, an antiserum is obtained after about 4 weeks or more. Then, a polyclonal antibody fraction (IgG fraction) can be harvested therefrom, for example, by affinity chromatography using protein A.

The polyclonal antibody obtained in this manner is examined for S/M binding selectivity. If its value is not less than 100, the polyclonal antibody can be directly used as S antibody in the method of the present invention. If the S/M binding selectivity is less than 100, polyclonal antibodies having cross reactivity with *Streptococcus mutans* (hereinafter also referred to as "cross-reactive antibodies") may be removed from the resulting polyclonal antibody (hereinafter also referred to as the "raw antibody").

Generally, a polyclonal antibody is a mixture of various antibodies. It is believed that antibodies reacting with *Streptococcus mutans* coexist in the polyclonal antibody obtained in the above-described manner, resulting in a reduction in overall S/M binding selectivity. Accordingly, the cross-reactive antibodies may be selectively separated or removed from the raw antibody until the overall S/M binding selectivity reaches 100 or greater.

However, the cross-reactive antibodies include antibodies having various binding abilities for *Streptococcus mutans* (i.e., ranging from ones having a high affinity constant therefor to ones having a low affinity constant therefor). In order to enhance the S/M binding selectivity of the raw antibody to 100 or greater, it is necessary to remove not only antibodies having a high affinity constant for *Streptococcus mutans*, but also antibodies having a low affinity constant therefor. In a method for removing cross-reactive antibodies by using cells of *Streptococcus mutans* directly, the present inventors have succeeded in enhancing the S/M binding selectivity to 100 or greater by carrying out an absorption treatment using cells of *Streptococcus mutans* added at a high proportion of not less than 40 (OD₆₀₀) to 1 mg of the raw antibody. According to investigations made by the present inventors, it has also been found that, in order to obtain an antibody having an S/M binding selectivity of the order of 50 or 90, cells may be added at a proportion of about 1 (OD₆₀₀) or about 4 (OD₆₀₀), respectively, to 1 mg of the raw material. This indicates that, in order to obtain an antibody having an S/M binding selectivity of not less than 100, a significantly larger quantity of cells than that usually required must be used for the raw antibody.

No particular limitation is placed on the method for removing cross-reactive antibodies from the raw antibody, but it is preferable to employ an affinity purification technique. When cross-reactive antibodies are removed according to an affinity purification technique, cells of *Streptococcus mutans* or cell surface antigens derived from the cells, which have been immobilized on an insoluble carrier, are brought into contact with the raw antibody. Thus, cross-reactive antibodies can be adsorbed to the insoluble carrier (also referred to as the "M antigen-immobilized carrier") and separated together with the insoluble carrier. Alternatively, without using an insoluble carrier, cells of *Streptococcus mutans* may be directly used and brought into contact with the raw antibody. Thus, cross-reactive antibodies can be adsorbed to the cells and separated together with the cells. In these methods, antibodies having a low binding ability, or no binding ability, for *Streptococcus mutans* are not adsorbed to the M antigen-immobilized carrier or the cells of *Streptococcus mutans*. Consequently, after the raw antibody is brought into contact with the carrier or the cells and then separated therefrom, its S/M binding selectivity is enhanced.

When an insoluble carrier is used, any of conventionally known insoluble carriers such as agarose, dextran, cellulose, polyacrylamide, polystyrene, vinyl chloride, glass, silicone rubber and porous silica beads may be used without any restriction. As the method for immobilizing cells of *Streptococcus mutans* or cell surface antigens derived from the cells to such carriers, any of chemical combination methods (e.g., the cyanogen bromide activation method) and methods utilizing physicochemical adsorption may be employed without any restriction.

The method using cells of *Streptococcus mutans* directly is preferred because it is simple in operation and a plurality of antibodies reacting with a plurality of antigens present on the cell surface of *Streptococcus mutans* can be removed at the same time. The cells used for this purpose can readily be prepared, for example, by culturing a reference strain of *Streptococcus mutans* in BHI liquid medium. As the cells, living cells may be used as they are, and dead cells prepared by heat treatment, formalin treatment or the like may also be used. As the reference strain of *Streptococcus mutans*, any of the serotype c, e and f strains may be used, and a mixture thereof may also be used. However, it is preferable to use a strain of serotype c or e. As described previously, in order to enhance the S/M binding selectivity of the raw antibody to 100 or greater and preferably 1,000 or greater, it is necessary to fully remove not only antibodies having a high affinity constant, but also antibodies having a low affinity constant. To this end, it is preferable to add cells at a proportion of not less than 40 (OD₆₀₀) to 1 mg of the raw antibody, as described above.

When the raw antibody is brought into contact with the aforesaid M antigen-immobilized carrier and then separated therefrom according to the affinity purification technique, there may be employed either of the so-called batch process and chromatographic process. Moreover, these processes may also be employed in combination.

In the chromatographic process, the raw antibody is added to a column packed with the M antigen-immobilized carrier. Then, a fraction flowing out without being retained therein may be collected. In the batch process, the raw antibody is mixed with a suspension of the M antigen-immobilized carrier to effect an antigen-antibody reaction to a full degree. Thereafter, the raw antibody may be separated, for example, by centrifuging the mixture and recovering the supernatant.

When cells of *Streptococcus mutans* are directly used, the raw antibody may be brought into contact with them and separated therefrom in the same manner as in the aforesaid batch process. That is, the raw antibody is mixed with a cell suspension to effect an antigen-antibody reaction to a full degree. Thereafter, the raw antibody may be separated, for example, by centrifuging the mixture and recovering the supernatant.

In the above-described manner, the present inventors obtained several polyclonal antibodies having an S/M binding selectivity of not less than 100. These polyclonal antibodies were produced by rabbits and named α6715, αB13 and α6715-B13.

As another procedure for separating cross-reactive antibodies according to the affinity purification technique, the raw antibody may be brought into contact with an insoluble carrier having immobilized thereto cell surface antigens specific for *Streptococcus sobrinus* (hereinafter also referred to as the "S antigen-immobilized carrier"), so as to cause an antibody having a high binding ability for *Streptococcus sobrinus to* be adsorbed thereto. Thereafter, the adsorbed antibody may be liberated by a conventionally know technique (e.g., by changing pH, ionic strength or permittivity) to obtain an antibody having an S/M binding selectivity of not less than 100. In this case, either of the batch process and the chromatographic process may preferably be used again.

Since the polyclonal antibody freed of cross-reactive antibodies may contain components arising from the bacterial cells, it is preferable to isolate an antibody fraction by further purification. As the purification technique, any of well-known techniques such as salting out, gel filtration, ion-exchange chromatography, affinity chromatography and electrophoresis may be employed without restriction. However, it is especially preferable to employ affinity chromatography using protein A, because it is simple in operation and an antibody fraction can be specifically recovered.

The various S antibodies obtained in the above-described manner may be used alone or as a mixture of antibodies having different properties. For example, an antibody whose reactivity with the serotype d strain of *Streptococcus sobrinus* is not less than twice the reactivity with the serotype g strain thereof may be suitably mixed with an antibody whose reactivity with the serotype g strain of *Streptococcus sobrinus is* not less than twice the reactivity with the serotype d strain thereof. Thus, there may obtained an antibody mixture having equal reactivity with the serotype d and g strains.

In the assay method of the present invention, the quantity of *Streptococcus sobrinus* present in test fluids and the quantity of *Streptococcus mutans* present therein can be simultaneously determined by the combined use of S antibody and an antibody combining specifically with *Streptococcus mutans* (hereinafter also referred to as "M antibody").

As used herein, the term "antibody combining specifically with *Streptococcus mutans* (M antibody)" means an antibody which exhibits a high binding ability for *Streptococcus mutans*, but only a binding ability below background level for other oral bacteria, particularly other oral streptococci including *Streptococcus sobrinus.* This term is not limited to an antibody of a particular globulin class, but comprehends antibodies of any presently known globulin classes. Moreover, it comprehends not only the ordinary antibody molecule, but also partial decomposition products of the antibody (e.g., Fab, Fab' and Fab'2), partial structures containing the active fragment of the antibody (i.e., the antigen-recognition site of the antibody), and the like.

Such antibodies are well known, and one specific example thereof is a monoclonal antibody described in Japanese Patent Laid-Open No. 36400/'98. However, no particular limitation is placed on the type of the antibody, provided that it meets the above-described property requirements. Thus, polyclonal and monoclonal antibodies prepared according to well-known procedures can be used without any restriction. For example, when the aforesaid antibody is a polyclonal antibody, it may preferably be obtained by immunizing an immune animal with whole cells of *Streptococcus mutans* or an antigenic extract obtained from the whole cells to produce a polyclonal antibody against *Streptococcus mutans*, and then freeing the polyclonal antibody of polyclonal antibodies having cross reactivity with other oral bacteria. The method for removing polyclonal antibodies having cross reactivity may be carried out in the same manner as the above-described affinity purification technique for preparing S antibody, except that bacteria other than *Streptococcus mutans* are used as adsorptive antigens.

Moreover, in the assay method of the present invention, the quantity of *Streptococcus sobrinus* present in test fluids and the total quantity of *Streptococcus mutans* and *Streptococcus sobrinus* present therein can be simultaneously determined by the combined use of S antibody and an antibody combining specifically with *Streptococcus mutans* and *Streptococcus sobrinus* (hereinafter also referred to as "MS antibody").

As used herein, the term "antibody combining specifically with *Streptococcus mutans* and *Streptococcus sobrinus* (MS antibody)" means an antibody which exhibits a high binding ability for *Streptococcus mutans* and *Streptococcus sobrinus*, but only a binding ability below background level for other oral bacteria. In order to determine the total quantity of *Streptococcus mutans* and *Streptococcus sobrinus* present in test fluids, it is preferable that the antibody have an equal binding ability for *Streptococcus mutans* and *Streptococcus sobrinus*. As used herein, the statement that the antibody has equal immunoreactivity (also referred to as binding ability or briefly as reactivity) for *Streptococcus mutans* and *Streptococcus sobrinus* means that, when an immune complex is detected according to a conventionally known immunoassay technique using the same quantity of *Streptococcus mutans* or *Streptococcus sobrinus* as the antigen, the mutual ratio between the detected values obtained from both bacteria is within 2 and preferably within 1.5.

As the antibody, polyclonal and monoclonal antibodies prepared according to well-known procedures can be used without any restriction, provided that they meets the above-described property requirements. Alternatively, a mixture of antibodies having different properties may also be used. For example, a mixture of the aforesaid M antibody and S antibody may be used after being adjusted so as to have equal reactivity for *Streptococcus mutans* and *Streptococcus sobrinus*.

No particular limitation is placed on the method for determining the quantity of *Streptococcus sobrinus* present in test fluids and, if necessary, the quantity of *Streptococcus mutans present* therein or the total quantity of *Streptococcus mutans* and *Streptococcus sobrinus* present therein according to an immunoassay technique using S antibody so prepared and, if necessary, M antibody and MS antibody so prepared. There may be used any of conventionally known immunoassay techniques for assaying an antigen through the medium of an antigen-antibody reaction, such as an immunoagglutination techniques, optical immunoassay techniques, labeled immunoassay techniques and combinations thereof. These immunoassay techniques can preferably be carried out by using an immunoassay reagent containing S antibody and, if necessary, M antibody and MS antibody. No particular limitation is placed on the form of the immunoassay reagent, provided that it contains S antibody and, if necessary, M antibody and MS antibody. Specifically, the immunoassay reagent may have any of various forms including a solution containing the aforesaid antibodies used for the reagent (i.e., constituent antibodies); an insoluble carrier (e.g., particles or a membrane) having the constituent antibodies immobilized thereto; a product obtained by combining the constituent antibodies with a labeling substance selected from radioactive materials, enzymes, various pigments, colloids and various particles, or a solution or suspension thereof; an insoluble carrier (e.g., particles or a membrane) having such labeled constituent antibodies immobilized thereto, or a suspension thereof; and combinations thereof.

Now, various assay techniques using such immunoassay reagents are described below.

### <Immunoagglutination techniques>

These techniques are methods for detecting and assaying *Streptococcus sobrinus* present in test fluids by utilizing the agglutination reaction of an insoluble carrier on the basis of an antigen-antibody reaction. Semiquantitative techniques include a latex agglutination technique, a microtiter technique and the like, and quantitative techniques include a latex assay technique and the like.

For example, in order to immunologically assay *Streptococcus sobrinus* present in test fluids according to a latex agglutination technique, an assay reagent composed of antibody-sensitized particles comprising latex beads having S antibody immobilized thereto (also referred to as S antibody-sensitized particles) is prepared. Then, an assay can be made by mixing this assay reagent with a test fluid to effect an antigen-antibody reaction and detecting the degree of agglutination of the sensitized particles by visual observation, optical measurement or the like.

In this procedure, an assay reagent composed of antibody-sensitized particles comprising latex beads having M antibody or MS antibody immobilized thereto (also referred to as M antibody-sensitized particles or MS antibody-sensitized particles, respectively) may be separately prepared. Then, a suspension of the aforesaid S antibody-sensitized particles and a suspension of the M antibody-sensitized particles or MS antibody-sensitized particles are placed in separate test tubes, and test fluids derived from an identical test fluid are added to the test tubes and mixed with the respective assay reagents to effect an antigen-antibody reaction. Thus, the quantity of *Streptococcus mutans* present in the same test fluid or the total quantity of *Streptococcus mutans* and *Streptococcus sobrinus* present therein can also be determined by detecting the degree of agglutination of each type of sensitized particles by visual observation, optical measurement or the like.

### <Optical immunoassay techniques>

These techniques are methods in which the presence or absence of an antigen-antibody reaction is optically detected. They include, for example, a technique in which, when an antigen-antibody reaction is effected by bringing a test fluid into contact with S antibody, a change in turbidity caused by an agglutination product resulting from the antigen-antibody reaction is detected; a technique in which, when an antigen-antibody reaction is effected by bringing a test fluid into contact with an assay reagent comprising S antibody immobilized on a transparent support, a change in transmittance caused by the reaction of particulate cells with the transparent support is detected; and a technique in which, when an antigen-antibody reaction is effected by bringing a test fluid into contact with a thin layer having S antibody immobilized thereto (hereinafter also referred to as the antibody layer), the resulting change in refractive index is detected as a change of transmitted light or surface plasmon waves.

### <Labeled immunoassay techniques>

According to these techniques, an antigen-antibody reaction is effected by bringing a test fluid into contact with a measuring reagent containing a labeled antibody obtained by reacting S antibody with any of various labeling substances such as radioactive materials, enzymes, various pigments, colloids, and various types of particles. Thereafter, *Streptococcus sobrinus* present in the test fluid can be detected and assayed by measuring the amount of labeling substance having reacted with *Streptococcus sobrinus* present in the test fluid (i.e., the radioactivity, enzyme activity, fluorescence intensity or color arising from the labeling substance). Alternatively, *Streptococcus sobrinus* present in a test fluid can also be detected and assayed by bringing the test fluid into contact with an assay reagent comprising an insoluble carrier (e.g., particles, a membrane or an ELISA plate) having S antibody immobilized thereto so as to effect an antigen-antibody reaction, bringing the assay reagent into contact with another assay reagent containing a labeled antibody comprising S antibody labeled with a labeling substance so as to further effect an antigen-antibody reaction, and then determining the amount of the labeling substance; or by mixing the test sample with *Streptococcus sobrinus* cells or cell surface antigens labeled with a labeling substance, bringing this mixture into contact with an assay reagent comprising an insoluble carrier having S antibody immobilized thereto so as to effect an antigen-antibody reaction, and then determining the amount of the labeling substance having reacted with S antibody.

Usable labeling substances include radioactive materials such as radioactive iodine and radioactive carbon; enzymes such as peroxidase, alkaline phosphatase and galactosidase; various pigments such as fluorescent pigments (e.g., fluorescein isothiocyanate and tetramethylrhodamine); colloids such as gold colloid and carbon colloid; and various types of particles such as colored latex particles. When enzyme labeling is employed, the antibody may be labeled with an enzyme, for example, either directly by the covalent reaction of a thiol group with a maleimide group or of an amino group with an aldehyde group, or indirectly through the medium of a biotin-avidin complex. Moreover, when alkaline phosphatase or peroxidase is used as the labeling enzyme and a chemiluminescent material (e.g., a dioxetane derivative for the former enzyme or a luminol derivative for the latter enzyme) is used as the substrate for the enzyme, the luminescence of the substrate may be used for purposes of detection.

As the labeled immunoassay technique, any of conventionally known techniques may be employed without any restriction, depending on the type of the label used. Among others, radioimmunoassay using a radioactive material as a label, enzyme immunoassay using an enzyme as a label, fluoroimmunoassay using a pigment (in particular, a fluorescent pigment) as a label, chemiluminescence immunoassay in which a chemiluminescent material serving as a substrate for an enzyme is utilized as a label, and the like have highly quantitative properties. Accordingly when a highly accurate quantitative assay is to be made, it is preferable to employ these techniques. Flow-through immunoassay, immunochromatographic assay and immunofiltration assay using a colloid or particulate material as a label, and latex agglutination assay are characterized by a simple operation.

Among these various immunoassay techniques, quantitative latex assay and enzyme immunoassay permit a large number of test fluids to be treated by means of an automatic analyzer, and are hence suitable for use as testing methods for mass examination. Flow-through immunoassay, immunochromatographic assay and latex agglutination assay are not only simple, but also permit rapid measurements without requiring any special knowledge or equipment. Thus, they can be carried out even in dental clinics and at home, and are hence suitable for use as general-purpose testing methods.

The operations, procedures and other conditions in these various labeled immunoassay techniques are not substantially different from those commonly employed in the art, and they may be carried out in substantially the same manner as the well-known noncompetitive assay technique, competitive assay technique, sandwich technique and the like. Moreover, in conjunction with the aforesaid antibody used in the present invention, substances capable of reacting with aforesaid antibody used in the present invention, such as a secondary antibody labeled by a wide variety of substances or protein A, may be used to detect and assay *Streptococcus sobrinus*.

Moreover, if necessary, a plurality of antibodies may be used in combination to detect and assay *Streptococcus sobrinus*. For example, in an immunoassay method based on the principle of the sandwich technique, a monoclonal antibody may be used as an antibody immobilized to a solid phase, and a polyclonal antibody may be used as a labeled antibody.

For example, when *Streptococcus sobrinus* present in a test fluid is assayed according to a flow-through immunoassay technique, an assay reagent may be prepared by immobilizing S antibody on a porous membrane so as to serve as an antibody for capturing *Streptococcus sobrinus*. Then, the test fluid is brought into contact with the surface of the porous membrane and made to pass through the porous membrane in a direction perpendicular to its surface, so that *Streptococcus sobrinus* present in the test fluid is captured on the porous membrane by an antigen-antibody reaction. Thereafter, another assay reagent comprising a solution of labeled S antibody is brought into contact with the surface of the porous membrane and made to pass through the porous membrane in a direction perpendicular to its surface. Thus, *Streptococcus sobrinus* present in the test fluid can be rapidly and simply assayed by examining the presence or absence, or amount, of the labeled antibody present on the porous membrane.

In the labeled immunoassay techniques, both the quantity of *Streptococcus sobrinus* present in test fluids and the quantity of *Streptococcus mutans* present therein or the total quantity of *Streptococcus mutans* and *Streptococcus sobrinus* present therein can simultaneously be determined by using an assay reagent containing S antibody and M antibody or MS antibody. Thus, similarly to assays according to a conventional cultivation process, the quantity of essentially all mutans streptococci existing in the oral cavity can be determined, making it possible to establish a comparison with the results obtained by conventional methods.

In order to make an assay by using the aforesaid assay reagent containing S antibody and M antibody or MS antibody, S antibody and M antibody or MS antibody are reacted with different labeling substances to form labeled antibodies, an assay reagent containing these labeled antibodies is prepared, and a test fluid is brought into contact with the assay reagent to effect an antigen-antibody reaction. Thereafter, the amount of labeling substance having reacted with *Streptococcus sobrinus* present in the test fluid and the amount of labeling substance having reacted with *Streptococcus mutans* present in the test fluid or the amount of labeling substance having reacted with both *Streptococcus mutans* and *Streptococcus sobrinus* are separately determined at the same time. Alternatively, there may be used an assay reagent prepared by immobilizing S antibody and M antibody or MS antibody at different positions of an identical insoluble carrier (e.g., a membrane or ELISA plate). After a test fluid is brought into contact with the assay reagent to effect an antigen-antibody reaction, another assay reagent comprising labeled S antibody and labeled M antibody or labeled MS antibody which are obtained by reacting S antibody and M antibody or MS antibody with a labeling substance are brought into contact therewith to further effect an antigen-antibody reaction, and the amount of labeling substance having reacted with the insoluble carrier is determined. Alternatively, a test fluid may be mixed with *Streptococcus sobrinus* cells or cell surface antigens which are labeled with a labeling substance, and *Streptococcus mutans* cells or cell surface antigens which are labeled with a labeling substance. This mixture is brought into contact with an assay reagent comprising an insoluble carrier having S antibody and M antibody or MS antibody immobilized thereto to effect antigen-antibody reactions. Thereafter, the amounts of labeling substances having reacted with S antibody and M antibody or MS antibody may be determined at the same time. In this process, separate insoluble carriers having each antibody immobilized thereto may be used in parallel, or an identical insoluble carrier having both antibodies immobilized at different positions thereof may be used.

For example, when the quantities of *Streptococcus sobrinus* and *Streptococcus mutans* present in a test fluid are simultaneous determined according to a flow-through immunoassay technique, an assay reagent may be prepared by immobilizing S antibody and M antibody at distinguishable positions on a porous membrane so that S antibody will serve as an antibody for capturing *Streptococcus sobrinus* and M antibody will serve as an antibody for capturing *Streptococcus mutans.* Then, the test fluid is brought into contact with the surface of the porous membrane and made to pass through the porous membrane in a direction perpendicular to its surface, so that *Streptococcus sobrinus* and *Streptococcus mutans* present in the test fluid are captured at the distinguishable positions on the porous membrane by antigen-antibody reactions. Thereafter, a solution of S antibody and M antibody which have been labeled with the same labeling substance or difference labeling substances is brought into contact with the surface of the porous membrane and made to pass through the porous membrane in a direction perpendicular to its surface. Thus, the quantities *of Streptococcus sobrinus* and *Streptococcus mutans* present in the test fluid can be rapidly and simply determined at the same time by simultaneously examining the presence or absence, or amounts, of the labeled antibodies present at the distinguishable positions on the porous membrane. Alternatively, by using an assay reagent containing S antibody and MS antibody, the quantity of *Streptococcus sobrinus* and the total quantity of *Streptococcus mutans* and *Streptococcus sobrinus* can simultaneously be determined in the same manner.

As described previously, the immunochromatographic technique is a method which can be carried out even in dental clinics and at home. The present inventors have succeeded in enhancing its detection limit by using S antibody in an assay reagent commonly used in this technique and called an immunochromatographic strip (also referred to briefly as a strip), and thereby assaying *Streptococcus sobrinus* present in test fluids with higher sensitivity. Consequently, the immunochromatographic technique using the aforesaid strip incorporating S antibody (hereinafter also referred to as the immunochromatographic technique of the present invention) can be an excellent general-purpose testing method for judging the degree of risk of dental caries. Now, this technique is more specifically explained with reference to the drawings.

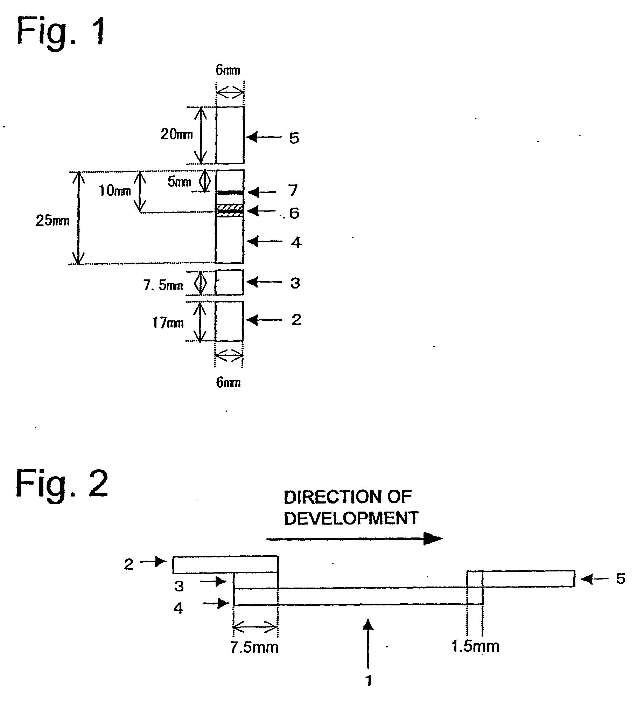

FIG. 2 illustrates the structure of a typical strip 1 which can preferably be used in the immunochromatographic technique of the present invention, and FIG. 1 is a schematic view of several components of this strip 1. Similarly to conventional strips, this strip 1 consists of several components comprising porous supports suited for their respective purposes. Specifically, they include a sample pad 2 for absorbing and holding a test fluid temporarily, a conjugate pad 3 for holding a labeled antibody temporarily, a development membrane 4 having a detection antibody immobilized thereto, and an absorption pad 5 for absorbing the test fluid developed from sample pad 2, these components being joined together in the order mentioned. In conjugate pad 3 of strip 1, an antibody for labeling *Streptococcus sobrinus* present in the test fluid (hereinafter referred to as the "labeled antibody"; for example, S antibody labeled with a labeling substance such as gold colloid may be used as the labeled antibody) is held by applying it to conjugate pad 3 and drying it. Moreover, at detection line 6 of the aforesaid development membrane 4, S antibody is immobilized as a detection antibody for capturing *Streptococcus sobrinus present* in the test fluid. It is to be understood that the dimensions (lengths) shown in FIGs. 1 and 2 are given for illustrative purposes only and the size of the strip used in the immunochromatographic technique of the present invention is not limited to the values shown in these figures.

No particular limitation is placed on the materials used for the porous supports constituting the aforesaid components, and they may be suitably selected from moisture-absorbing materials, porous materials, fibrous materials and the like according to the respective purposes of the components. For example, filter paper, blotting paper and the like are preferably used for sample pad 2, and glass fiber cloth, polypropylene nonwoven fabric, glass filters and the like are preferably used for conjugate pad 3. Moreover, nitrocellulose, nitrocellulose containing cellulose acetate, and the like are preferably used for development membrane 4, and filter paper, blotting paper and the like are preferably used for absorption pad 5.

No particular limitation is placed on the method for immobilizing a detection antibody and a control antibody to development membrane 4, and conventionally known physical adsorption methods and covalent reaction methods may be used without any restriction. Alternatively, they may be immobilized after being mixed with other proteins or the like. In order to suppress the nonspecific adsorption of test fluid components to the development membrane or improve the wettability of the development membrane by the test fluid, the development membrane having the antibodies immobilized thereto may be subjected to a blocking treatment with a protein, lipid, polymeric compound or the like according to a well-known technique. Although no particular limitation is placed on the protein or other substance used for this blocking treatment, it is preferable to use BSA, skim milk and like substances which are used in order to suppress nonspecific reactions in common immunoassay methods. Moreover, in order to regulate the water absorption properties of the development membrane and thereby allow the test fluid to be uniformly developed through the development membrane, the surfaces of the development membrane may be coated or impregnated with a hydrophilic polymer or surface-active agent.

As to conjugate pad 3, it is preferable to block the conjugate pad with a water-soluble polymer or a sugar (e.g., saccharose) in advance, added the labeled antibody thereto, and dry it or to mix the labeled antibody with a water-soluble polymer or a sugar (e.g., saccharose) in advance, apply this mixture to the conjugate pad, and dry it. Thus, when a test fluid is added, a conjugate of *Streptococcus sobrinus* and the labeled antibody can be easily released from this component. As the aforesaid water-soluble polymer, there may be used polyvinyl pyrrolidone, polyvinyl alcohol, polyethylene glycol, cellulose ethers (e.g., methyl cellulose, ethyl cellulose, carboxymethyl cellulose, carboxyethyl cellulose, oxyethyl cellulose and cyanoethyl cellulose), gelatin and the like.

As the labeling substance for preparing the labeled antibody, there may be used not only labeling substances recognizable by visual observation, such as gold colloid, carbon colloid and colored latex, but also radioactive materials and fluorescent materials. Alternatively, it is also possible to use an enzyme as the labeling substance, add a substrate after the completion of an antigen-antibody reaction, and detect a compound formed by the enzyme reaction. No particular limitation is placed on the method for labeling the antibody, and there may be employed any of various methods which are commonly employed in the prior art.

In the immunochromatographic technique of the present invention, a test fluid is added to sample pad 2 disposed adjacent to conjugate pad 3 of the aforesaid strip 1. Then, the strip is allowed to stand at room temperature for 1 to 30 minutes so as to develop the labeled antibody, together with the test fluid, to detection line 6. Consequently, on the principle of the sandwich technique, *Streptococcus sobrinus* having reacted with the labeled antibody in the developed fluid is captured on detection line 6 by the detection antibody. Thus, the presence or absence, or quantity of *Streptococcus sobrinus present* in the test fluid can be determined by detecting the labeled antibody combined with the captured *Streptococcus sobrinus*. Moreover, an antibody reacting with the labeled antibody is immobilized at control judgment line 7 located upstream of the aforesaid detection line 6. Thus, by detecting the labeled antibody at this position, it can be confirmed that the labeled antibody has been normally developed together with the test fluid. It is to be understood that the positions, order, shapes and number of detection line 6 and control judgment line 7 on development membrane 1 shown in FIG. 1 are given for illustrative purposes only, and the present invention is not limited thereto.

Having described the immunochromatographic technique using a strip onto which a labeled antibody has previously be applied and dried, an assay may also be carried out according to a procedure in which a labeled antibody is previously mixed with a test fluid to effect an antigen-antibody reaction and the resulting mixture is applied to one end of a porous support having a detection antibody immobilized thereto.

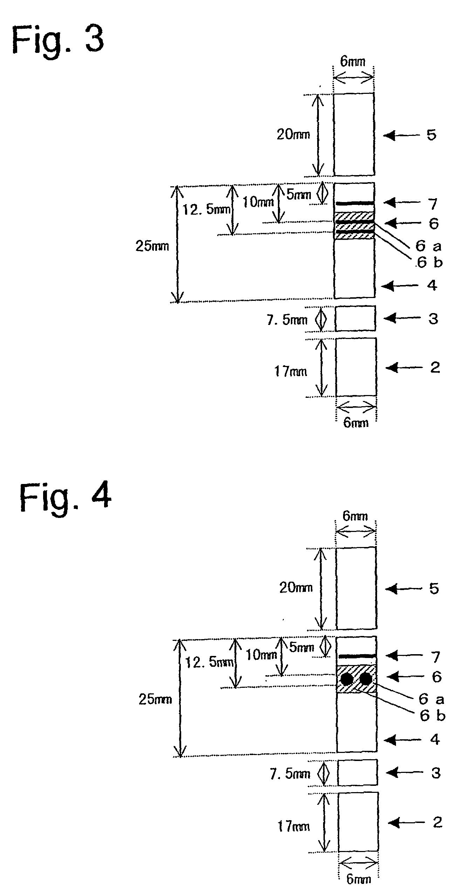

Moreover, in the immunochromatographic technique of the present invention, labeled antibodies comprising S antibody and M antibody which have been labeled with a labeling substance such as gold colloid (labeled S antibody and labeled M antibody) may be held on a conjugate pad 3 as shown in FIG. 3 by applying the antibodies to the pad and drying them, and detection antibodies comprising S antibody and M antibody may be immobilized at detection lines 6a and 6b, respectively, on the aforesaid development membrane 4. By using a strip so formed, the quantities of *Streptococcus sobrinus* and *Streptococcus mutans present* in a test fluid can be simultaneously determined on the same principle as described above. In the embodiment shown in FIG. 3, detection lines 6a and 6b are arranged in series and in the form of lines (or bands), so as to be located at different distances from the downstream end of development membrane 4 toward which the test fluid is developed. However, no particular limitation is placed on the position at which each detection antibody is immobilized, and the size and shape of the immobilization area. For example, as shown in FIG. 4, S antibody and M antibody may be arranged in parallel and in the form of circles or spots, at the position of detection line 6 of development membrane 4 so as to be located at the same distance from the downstream end of development membrane 4 toward which the test fluid is developed. Moreover, the areas in which both antibodies are immobilized need not be completely separated, but they may have any desired shapes and positional relationship that permit them to be easily distinguished by visual observation, such as mutually crossed shapes.

Furthermore, in each strip using a development membrane 4 as described above in connection with FIGs. 3 and 4, MS antibody may be used in place of the M antibody used as a detection antibody and a labeled antibody. Thus, the quantity of *Streptococcus sobrinus* present in a test fluid and the total quantity of *Streptococcus sobrinus* and *Streptococcus mutans* present therein can be simultaneously determined.

An assay based on the immunochromatographic technique of the present invention may be carried out, for example, according to the following procedure. Thus, it is possible to determine the quantity of *Streptococcus sobrinus* present in the oral cavity of a human subject rapidly and simply, and thereby diagnose or judge the degree of risk of dental caries.

Specifically, first of all, ① dental plaque or saliva is collected from the oral cavity (by using a spatula or swab for dental plaque or a dropper for saliva). ② When saliva is collected, it is directly used or suspended in a sample diluent to prepare a test fluid. When dental plaque is collected, a test fluid is prepared by suspending the dental plaque in a sample diluent and, if necessary, subjecting the resulting suspension to a plaque dispersion treatment such as ultrasonication. ③ An aliquot (e.g., 100-200 µl) of the test fluid is taken and applied to the sample pad of the aforesaid strip. After the strip is allowed to stand for a predetermined time (e.g., 1-30 minutes), the presence or absence, or quantity, of *Streptococcus sobrinus* can be judged on the basis of the presence or absence of coloring, or degree of coloring, of detection line 6 and control judgment line 7 of the strip.

For example, when test fluids prepared in the manner described in the examples which will be given later are assayed by using gold colloid as a labeling substance, the degree of coloring of detection line 6 with gold colloid varies according to the concentration of *Streptococcus sobrinus* in the test fluid, as shown in Table 1. Consequently, the degree of risk of dental caries can be diagnosed or judged on the basis of the criteria shown in Table 2. In Table 2, the criteria are expressed in terms of the cell concentrations of test fluids prepared at specific dilution ratios. However, it is a matter of course that the cell concentrations per unit amount of dental plaque or saliva may be calculated from the dilution ratios used in the preparation of test fluids and used as criteria for judgment.

**Table 1**

| | | | | |
|---|---|---|---|---|
| Concentration of *S. sobrinus* (cells/ml) | 10⁷ | 10⁶ | 10⁵ | <10⁵ |
| Color of detection line | Dark red | Red | Light red | Colorless |
| Rating | +++ | ++ | + | - |

**Table 2**

| Detection line | Control line | Rating | Degree of risk of dental caries |
|---|---|---|---|
| Dark red to red | Red to dark red | +++ - + + | High risk |
| Red to light red | Red to dark red | ++ - + | Medium risk |
| Light red | Red to dark red | + | Low risk |
| Colorless | Red to dark red | - | No risk |
| Light red to dark red | Colorless | Retest | Undeterminable |
| Colorless | Colorless | Retest | Undeterminable |

When assays were carried out in the same manner as described above, but using a strip as shown in FIG. 3 in which M antibody is also incorporated and gold colloid is used as a labeling substance, the degree of coloring of detection lines 6a and 6b with gold colloid varies according to the concentrations of *Streptococcus sobrinus* and *Streptococcus mutans* in the test fluid, as shown in Table 3. Consequently, on the basis of the criteria shown in Table 4, the degree of risk of dental caries can be diagnosed or judged with higher accuracy. In FIG. 4, the indexes (AAA to D) indicating the degrees of risk of dental caries mean "AAA-A: high risk", "BB-B: medium risk", "C: low risk", and "D: no risk".

**Table 3**

| *S. sobrinus* | | | | |
|---|---|---|---|---|
| Concentration of *S. sobrinus* (cells/ml) | 10⁷ | 10⁶ | 10⁵ | <10⁵ |
| Color of detection line | Dark red | Red | Light red | Colorless |
| Rating | +++ | ++ | + | - |

| *S. mutans* | | | | |
|---|---|---|---|---|
| Concentration of *S. mutans* (cells/ml) | 10⁷ | 10⁶ | 10⁵ | 10⁵ |
| Color of detection line | Dark red | Red | Light red | Colorless |
| Rating | +++ | ++ | + | - |

**Table 4**

| | | Rating for *S. sobrinus* | | | |
|---|---|---|---|---|---|
| | | +++-++ | ++-+ | + | - |
| Rating for *S. mutans* | +++-++ | AAA | AA | A | BB |
| | ++ - + | AA | A | BB | B |
| | + | A | BB | B | C |
| | - | A | B | C | D |

When assays were carried out in the same manner as described above, but using a strip as shown in FIG. 3 in which MS antibody is also incorporated and gold colloid is used as a labeling substance, the degree of coloring of detection lines 6a and 6b with gold colloid varies according to the concentrations of *Streptococcus sobrinus* and *Streptococcus mutans* in the test fluid, as shown in Table 5. Consequently, on the basis of the criteria shown in Table 6, the degree of risk of dental caries can be diagnosed or judged with higher accuracy. In Table 6, the indexes (AAA to D) indicating the degrees of risk of dental caries are the same as those shown in Table 4.

Thus, the quantity of *Streptococcus sobrinus* which is especially important as a bacterium responsible for dental caries, and if necessary, the quantity of *Streptococcus mutans* or the total quantity of *Streptococcus sobrinus* and *Streptococcus mutans*, can be rapidly and simply determined according to the immunochromatographic technique, so that the degree of risk of dental caries in the subject can be diagnosed or judged.

**Table 5**

| *S. sobrinus* | | | | |
|---|---|---|---|---|
| Concentration of *S. sobrinus* (cells/ml) | 10⁷ | 10⁶ | 10⁵ | <10⁵ |
| Color of detection line | Dark red | Red | Light red | Colorless |
| Rating | +++ | ++ | + | - |

| *S. mutans* and *S. sobrinus* | | | | |
|---|---|---|---|---|
| Total concentration of *S. mutans* and *S. sobrinus* (cells/ml) | 10⁷ | 10⁶ | 10⁵ | <10⁵ |
| Color of detection line | Dark red | Red | Light red | Colorless |
| Rating | +++ | ++ | + | - |

**Table 6**

| | | Rating for *S. sobrinus* | | | |
|---|---|---|---|---|---|
| | | +++ - + + | ++ - + | + | - |
| Rating for *S. mutans* and *S. sobrinus* | +++ - + + | AAA | AA | A | BB |
| | ++ - + | | A | BB | B |
| | + | | | B | C |
| | - | | | | D |

The present invention is more specifically explained with reference to the following examples. However, these examples are not to be construed to limit the scope of the invention.

### Preparation Example 1

### Preparation of a polyclonal antibody against Streptococcus sobrinus and evaluation of the reactivity of the antibody

### (1) Preparation of cell sample suspensions

BHI liquid medium was prepared by dissolving 3.7 g of BHI (DIFCO) in 100 mg of ultrapure water and autoclaving the resulting solution. 6715 (*Streptococcus sobrinus*, serotype g) was suspended in 2 ml of BHI liquid medium and incubated at 37°C for 5 hours under anaerobic conditions (N₂:H₂:CO₂=80:10:10). Thereafter, the culture medium was centrifuged at 4,000g for 5 minutes and the supernatant comprising medium components was removed to recover the precipitate of bacterial cells. Then, the precipitate was suspended in 5 ml of PBS and centrifuged under the same conditions. The precipitate was washed by repeating this procedure three times. Subsequently, a cell sample suspension was prepared by suspending the resulting precipitate of bacterial cells in PBS and adjusting the suspension to A₆₀₀=1.0. After this cell sample suspension was ultrasonicated and suitably diluted, it was spread over a plate of BHI medium. The colonies so formed was counted and the resulting count was multiplied by the dilution ratio of the cell sample suspension. Thus, the cell concentration of the cell sample suspension was found to be about 1x10⁹ cells/ml.

According to substantially the same procedure, cell sample suspensions were prepared by using B13 (*Streptococcus sobrinus*, serotype d), Ingbritt (*Streptococcus mutans,* serotype c), P4 (*Streptococcus mutans*, serotype e), OMZ175 *(Streptococcus mutans,* serotype f), ATCC10556 (*Streptococcus sanguis*), IFO14252 (*Streptococcus salivarius*), ATCC49456 (*Streptococcus mitis*), ATCC35037 (*Streptococcus oralis*) and Charis *(Streptococcus gordonii*). In this list, 6715, B13, Ingbritt, Charis and the like are the names of reference strains, and ATCC10556, IFO14252 and the like are the deposition numbers of reference strains.

### (2) Preparation of an antiserum against Streptococcus sobrinus

6715 (*Streptococcus sobrinus*, serotype g) was suspended in 100 ml of autoclaved BHI liquid medium and incubated at 37°C for 5 hours under anaerobic conditions (N₂:H₂:CO₂=80:10:10). Thereafter, the culture medium was centrifuged at 4,000g for 5 minutes and the supernatant comprising medium components was removed to recover the precipitate of bacterial cells. Then, the precipitate was suspended in 100 ml of PBS and centrifuged under the same conditions. The precipitate was washed by repeating this procedure three times. The precipitate of bacterial cells was recovered, weighed and used to prepare a 6715 cell suspension having a concentration of 10 mg(wet cells)/ml. Then, an antigenic cell suspension was formed by diluting the above cell suspension twofold with 0.5% formalin-PBS so as to give a concentration of 5 mg(wet cells)/ml, and preserved at 4°C in the suspended state.

During a first week, 1 ml each of the antigenic cell suspension was injected three times into an auricular vein of a rabbit on alternate days. The same immunization treatment was repeated six times during second and third weeks. Furthermore, 0.5 ml of the antigenic cell suspension was mixed with an equal amount of an adjuvant, and this mixture was subcutaneously injected twice into the rabbit. One week after the final immunization, and after an increase in titer was confirmed by observing the degree of cell agglutination on a slide glass, blood was collected in the usual manner and an antiserum against *Streptococcus sobrinus* was obtained therefrom.

### (3) Purification of a polyclonal antibody against Streptococcus sobrinus

According to the procedure of step (1), 10 ml of a cell suspension containing Ingbritt (*Streptococcus mutans,* serotype c), P4 (*Streptococcus mutans,* serotype e) and OMZ175 (*Streptococcus mutans,* serotype f), each at a concentration of about 2x10¹⁰ cells/ml, was prepared.

Then, this cell suspension was mixed with 0.5 ml of the antiserum obtained in step (2), and the resulting mixture was reacted at 4°C for 60 minutes. After this mixture was centrifuged at 4,000g for 5 minutes, the supernatant was separated and filtered through a 0.22 µm filter.

Subsequently, the supernatant sample was added to a column packed with 1 ml of protein A-Sepharose (Pharmacia) pre-equilibrated with PBS. The column was washed with 5 ml of PBS and then eluted with 5 ml of a 0.1 M glycine-HCl buffer solution (pH 3.0). Immediately after that, the eluate was adjusted to pH 7.4 by the addition of 100 mM Tris-HCl (pH 9.0). The eluate of an IgG fraction was confirmed by measuring A₂₈₀. About 4 mg of IgG was recovered from 0.5 ml of the antiserum.

### (4) Evaluation of the polyclonal antibody against Streptococcus sobrinus by direct ELISA

The reactivity of the polyclonal antibody against *Streptococcus sobrinus* (hereinafter also referred to as "α6715") purified in the above step (3) with various bacterial strains was evaluated according to the following direct ELISA process.

Specifically, first of all, the various cell sample suspensions obtained in the above step (1) were diluted with a 0.1 M carbonate buffer solution (pH 9.0) so as to give concentrations of 1x10⁴ to 10⁹ cells/ml. 50 µl each of these diluted cell suspensions were added to wells of a 96-well immunoplate (Nunc; Maxisorp) and immobilized by allowing the immunoplate to stand at 4°C for 12 hours. Thereafter, the immunoplate was freed of the cell suspensions, and washed three times with 300 µl portions of PBS.

Then, 300 µl of a 2% BSA-0.1 M carbonate buffer solution (pH 9.0) was added to each well of the immunoplate, and allowed to stand at 37°C for 2 hours. Thereafter, the immunoplate was freed of the 2% BSA-carbonate buffer solution (pH 9.0), and washed three times with 300 µl portions of 0.05% Tween 20-PBS (pH 7.4).

Then, α6715 was diluted with 1% BSA-0.05% Tween 20-PBS (pH 7.4) so as to give a concentration of 1.0 µg/ml. 50 µl of this solution was added to each well of the immunoplate, and allowed to stand at 37°C for 1 hour. Thereafter, the immunoplate was freed of the solution, and washed three times with 300 µl portions of 0.05% Tween 20-PBS (pH 7.4).

Then, an alkaline phosphatase-labeled anti-rabbit IgG(Fc) polyclonal antibody (goat) (Cappel) was diluted with 1% BSA-0.05% Tween 20-PBS (pH 7.4) so as to give a concentration of 10 µg/ml. 50 µl of this solution was added to each well of the immunoplate, and allowed to stand at 37°C for 1 hour. Thereafter, the immunoplate was freed of the solution, and washed three times with 300 µl portions of 0.05% Tween 20-PBS (pH 7.4).

Then, 50 µl of a color-producing substrate solution comprising an aqueous 2-ethanolamine solution of p-nitrophenyl phosphate (BIORAD) was added to each well, and reacted at room temperature for 20 minutes. After completion of the reaction, the reaction was stopped by adding 50 µl of 0.4 M NaOH to each well, and the absorbance at 405 nm was measured. The results thus obtained are shown in Table 7.

**Table 7**

| Strain | Species/serotype | Quantity (cells/well) | A₄₀₅ |
|---|---|---|---|
| B13 | *Streptococcus sobrinus*/d | 5×10² | 0.005 |
| B13 | *Streptococcus sobrinus*/d | 5×10³ | 0.011 |
| B13 | *Streptococcus sobrinus*/d | 5×10⁴ | 0.062 |
| B13 | *Streptococcus sobrinus*/d | 5×10⁵ | 0.640 |
| B13 | *Streptococcus sobrinus*/d | 5×10⁶ | 1.635 |
| B13 | *Streptococcus sobrinus*/d | 5×10⁷ | >2.000 |
| 6715 | *Streptococcus sobrinus*/g | 5×10² | 0.004 |
| 6715 | *Streptococcus sobrinus*/g | 5×10³ | 0.013 |
| 6715 | *Streptococcus sobrinus*/g | 5×10⁴ | 0.080 |
| 6715 | *Streptococcus sobrinus*/g | 5×10⁵ | 0.653 |
| 6715 | *Streptococcus sobrinus*/g | 5×10⁶ | 1.682 |
| 6715 | *Streptococcus sobriaus*/g | 5×10⁷ | >2.000 |
| Ingbritt | *Streptococcus mutans*/c | 5×10⁵ | 0.005 |
| Ingbritt | *Streptococcus mutans*/c | 5×10⁶ | 0.004 |
| Ingbritt | *Streptococcus mutans*/c | 5×10⁷ | 0.012 |
| P4 | *Streptococcus mutans*/e | 5×10⁵ | 0.004 |
| P4 | *Streptococcus mutans*/e | 5×10⁶ | 0.003 |
| P4 | *Streptococcus mutans*/e | 5×10⁷ | 0.010 |
| OMZ175 | *Streptococcus mutans*/f | 5×10⁵ | 0.004 |
| OMZ175 | *Streptococcus mutans*/f | 5×10⁶ | 0.006 |
| OMZ175 | *Streptococcus mutans*/f | 5×10⁷ | 0.013 |
| ATCC10556 | *Streptococcus sanguis* | 5×10⁷ | 0.004 |
| IFO14252 | *Streptococcus salivarius* | 5×10⁷ | 0.003 |
| ATCC49456 | *Streptococcus mitis* | 5×10⁷ | 0.004 |
| ATCC35037 | *Streptococcus oralis* | 5×10⁷ | 0.006 |
| Charis | *Streptococcus gordonii* | 5×10⁷ | 0.007 |

### (5) Evaluation of the polyclonal antibody against Streptococcus sobrinus by competitive ELISA

The reactivity of α6715 with various bacterial strains was also evaluated according to the following competitive ELISA process.

Specifically, first of all, an immunoplate having bacterial cells immobilized thereto and blocked with BSA was prepared in the same manner as in the above step (4). Then, 50 µl each of 1% BSA-0.05% Tween 20-PBS (pH 7.4) solutions containing 1.0 µg/ml of α6715 and 10⁵ to 10⁹ cells/ml of *Streptococcus mutans* or *Streptococcus sobrinus* were added to wells of the immunoplate, and allowed to stand for 37°C for 1 hour. Thereafter, the immunoplate was freed of the solutions and washed three times with 300 µl portions of 0.05% Tween 20-PBS (pH 7.4).

Then, an alkaline phosphatase-labeled anti-rabbit IgG antibody was added in the same manner as in the above step (4). Thereafter, a color-producing substrate solution was added, and the absorbance at 405 nm was measured. The results thus obtained are shown in Table 8.

**Table 8**

| Quantity of 6715 immobilized (cells/well) | Strain added | Quantity of strain added (cells/well) | A₄₀₅ |
|---|---|---|---|
| 5×10⁵ | 6715 | 5×10⁷ | 0.018 |
| 5×10⁵ | 6715 | 5×10⁶ | 0.078 |
| 5×10⁵ | 6715 | 5×10⁵ | 0.184 |
| 5×10⁵ | 6715 | 5×10⁴ | 0.309 |
| 5×10⁵ | 6715 | 5×10³ | 0.325 |
| 5×10⁵ | 6715 | 0 | 0.320 |
| 5×10⁵ | Ingbritt | 5×10⁷ | 0.293 |
| 5×10⁵ | Ingbritt | 5×10⁶ | 0.325 |
| 5×10⁵ | Ingbritt | 5×10⁵ | 0.319 |
| 5×10⁵ | Ingbritt | 5×10⁴ | 0.321 |
| 5×10⁵ | Ingbritt | 5×10³ | 0.320 |
| 5×10⁵ | Ingbritt | 0 | 0.319 |

It can be seen from Table 7 that, when an antigen-antibody reaction is detected by using *Streptococcus sobrinus* and *Streptococcus mutans* as antigens in an identical quantity in the range of 5x10⁵ cells/ml to 5x10⁷ cells/well, the degree of reaction detected by using *Streptococcus sobrinus* as the antigen is not less than 100 times the degree of reaction detected by using *Streptococcus mutans* as the antigen. Moreover, it can also be seen that, since the quantity of *Streptococcus sobrinus* giving the same degree of reaction as that detected by using 5x10⁷ cells/well of *Streptococcus mutans* as the antigen is less than 5x10⁴ cells/well, the reactivity of α6715 with *Streptococcus sobrinus* is not less than 1,000 times its reactivity with *Streptococcus mutans*.

Furthermore, it can be seen from Table 8 that, since the inhibitory reaction detected by adding 5x10⁷ cells/well of *Streptococcus mutans* is equal to the inhibitory reaction detected by adding less than 5x10⁵ cells/well of *Streptococcus sobrinus,* the reactivity with *Streptococcus sobrinus* is not less than 100 times the reactivity with *Streptococcus mutans.*

### Example 1

### Preparation and evaluation of immunochromatographic strips for the assay of Streptococcus sobrinus

### (1) Preparation of a gold colloid-labeled polyclonal antibody against Streptococcus sobrinus

88 µl of 100 mM K₂CO₃ was added to 10 ml of a commercially available gold colloid solution (EY Laboratory) having a colloidal particle diameter of 40 nm. The resulting solution was adjusted to pH 9.0 and filtered through a 0.22 µm filter. When the absorbance of the gold colloid solution was measured at 520 nm, A₅₂₀ was found to be 1.0.

Then, 64 µl of a 2 mM borate buffer solution (pH 9.0) of α6715 adjusted to a concentration of 1 mg/ml was added to the above gold colloid solution with stirring, followed by standing at room temperature for 5 minutes. Then, 1.1 ml of a 10% skim milk-2 mM borate buffer solution (pH 9.0) was added thereto with stirring (to a final skim milk concentration of 1%), followed by standing at room temperature for 30 minutes. Then, the reacted solution was centrifuged at 10,000g for 30 minutes at 10°C. After the supernatant was removed, 2 ml of 2 mM PBS (pH 7.4) was added and the lower gold colloid fraction was resuspended therein. When the absorbance of the resuspended fraction was measured at 520 nm, A₅₂₀ was found to be 4.9. The resulting gold colloid fraction (hereinafter also referred to as the "gold colloid-labeled α6715") was preserved at 4°C.

### (2) Preparation of an immunochromatographic strip

At a detection line 6 and a control judgment line 7 formed on a development membrane 4 comprising a nitrocellulose membrane (MILLIPORE; High-Flow Membrane, SN, 25 mm x 6 mm), 1 µl of α6715 and anti-rabbit IgG(H+L) polyclonal antibody each having a concentration of 1 mg/ml were spotted, respectively. Then, the membrane was dried in an incubator at 37°C for 60 minutes to immobilize the antibodies thereto. This antibody-immobilized membrane was shaken in an aqueous 1% skim milk-0.01% Triton X100 solution at room temperature for 5 minutes. Then, the membrane was shaken in a 10 mM phosphate buffer solution (pH 7.4) at room temperature for 10 minutes. Thereafter, the membrane was taken out and dried in a dessicator for 60 minutes under aspiration with a vacuum pump.

Moreover, a conjugate pad 3 (MILLIPORE; 7.5 mm x 6 mm) was shaken in an aqueous 0.5% PVA-0.5% sucrose solution for 1 minute. Thereafter, the conjugate pad was taken out and dried in a dessicator for 60 minutes under aspiration with a vacuum pump. After 25 µl of gold colloid-labeled α6715 adjusted to A₅₂₀=1.0 was added to the conjugate pad, it was dried in a dessicator for 60 minutes under aspiration with a vacuum pump. Furthermore, a sample pad 2 (MILLIPORE; 17 mm x 6 mm) was shaken in an aqueous 0.05% Tween 20-PBS solution for 1 minute. Thereafter, the sample pad was taken out and dried in a dessicator for 60 minutes under aspiration with a vacuum pump. An absorption pad 5 (MILLIPORE; 20 mm x 6 mm) was used in its as-received condition.

The immunochromatographic strip components as shown in FIG. 1, which were prepared in the above-described manner, were placed on a plastic support and assembled into an immunochromatographic strip as shown in FIG. 2.

### (3) Evaluation of the specificity and sensitivity of immunochromatographic strips

Cells of various reference strains were suspended in PBS, and 100 µl of each suspension was added to sample pad 2 of each immunochromatographic strip. After 10 minutes, the presence or absence of a spot was judged. Specifically, The sensitivity and quantifying ability of the immunochromatographic strips were evaluated by examining the amount of gold colloid captured on the immobilized antibody spot by visual observation and rating it in four grades (+++: strongly positive; ++: positive; +: weakly positive; -: negative) according to the criteria shown in Table 1 above. The results thus obtained are shown in Table 9.

**Table 9**

| Strain | Species/serotype | Cell concentration of test fluid (cells/ml) | Rating |
|---|---|---|---|
| B13 | *Streptococcus sobrinus*/d | 10⁷ | +++ |
| B13 | *Streptococcus sobrinus*/d | 10⁶ | ++ |
| B13 | *Streptococcus sobrinus*/d | 10⁵ | + |
| B13 | *Streptococcus sobrinus*/d | 10⁴ | - |
| 6715 | *Streptococcus sobrinus*/g | 10⁷ | +++ |
| 6715 | *Streptococcus sobrinus*/g | 10⁶ | ++ |
| 6715 | *Streptococcus sobrinus*/g | 10⁵ | *+* |
| 6715 | *Streptococcus sobrinus*/g | 10⁴ | - |
| Ingbritt | *Streptococcus mutans*/c | 10 | - |
| P4 | *Streptococcus mutans*/e | 10⁹ | - |
| OMZ175 | *Streptococcus mutans*/f | 10⁹ | - |
| ATCC10556 | *Streptococcus sanguis* | 10⁹ | - |
| IFO14252 | *Streptococcus salivarius* | 10⁹ | - |
| ATCC49456 | *Streptococcus mitis* | 10⁹ | |
| ATCC35037 | *Streptococcus oralis* | 10 | - |
| Charis | *Streptococcus gordonii* | 10⁹ | - |

### Comparative Example 1

### Evaluation of a polyclonal antibody against Streptococcus sobrinus which was not subjected to an absorption treatment with Streptococcus mutans

First of all, an antiserum against *Streptococcus sobrinus* was prepared in the same manner as in step (2) of Preparation Example 1. This antiserum was not subjected to an absorption treatment with cells of *Streptococcus mutans*, but treated with a protein A column according to the procedure described in step (3) of Preparation Example 1. Thus, an IgG fraction (hereinafter also referred to as the "unpurified polyclonal antibody") was prepared therefrom. Then, according to the procedure described in step (4) of Preparation Example 1, the reactivity of the unpurified polyclonal antibody with various bacterial strains was evaluated by direct ELISA. The results thus obtained are shown in Table 10.

**Table 10**

| Strain | Species/serotype | Quantity (cells/well) | A₄₀₅ |
|---|---|---|---|
| B13 | *Streptococcus sobrinus*/d | 10⁵ | 0.675 |
| B13 | *Streptococcus sobrinus*/d | 10⁶ | 1.689 |
| B13 | *Streptococcus sobrinus*/d | 10⁷ | >2.000 |
| 6715 | *Streptococcus sobrinus*/g | 10⁵ | 0.687 |
| 6715 | *Streptococcus sobrinus*/g | 10⁶ | 1.712 |
| 6715 | *Streptococcus sobrinus*/g | 10⁷ | >2.000 |
| Ingbritt | *Streptococcus mutans*/c | 10⁶ | 0.206 |
| Ingbritt | *Streptococcus mutans*/c | 10⁶ | 0.521 |
| Ingbritt | *Streptococcus mutans*/c | 10⁷ | 0.823 |
| P4 | *Streptococcus mutans*/e | 10⁵ | 0.184 |
| P4 | *Streptococcus mutans*/e | 10⁶ | 0.460 |
| P4 | *Streptococcus mutans*/e | 10⁷ | 0.735 |
| OMZ175 | *Streptococcus mutans*/f | 10⁶ | 0.128 |
| OMZ175 | *Streptococcus mutans*/f | 10⁶ | 0.316 |
| OMZ175 | *Streptococcus mutans*/f | 10⁷ | 0.494 |
| ATCC10556 | *Streptococcus sanguis* | 10⁷ | 0.008 |
| IFO14252 | *Streptococcus salivarius* | 10⁷ | 0.010 |
| ATCC49456 | *Streptococcus mitis* | 10⁷ | 0.007 |
| ATCC35037 | *Streptococcus oralis* | 10⁷ | 0.012 |
| Charis | *Streptococcus gordonii* | 10⁷ | 0.014 |

Moreover, a gold colloid-labeled antibody was prepared according to the procedure of Example 1. Thereafter, immunochromatographic strips were prepared and their reactivity with various bacterial strains was evaluated. The results thus obtained are shown in Table 11.

**Table 11**

| Strain | Species/serotype | Cell concentration of test fluid (cells/ml) | Rating |
|---|---|---|---|
| B13 | *Streptococcus sobrinus*/d | 10⁷ | +++ |
| B13 | *Streptococcus sobrinus*/d | 10⁶ | ++ |
| B13 | *Streptococcus sobrinus*/d | 10⁵ | + |
| B13 | *Streptococcus sobrinus*/d | 10⁴ | - |
| 6715 | *Streptococcus sobrinus*/g | 10⁷ | +++ |
| 6715 | *Streptococcus sobrinus*/g | 10⁶ | ++ |
| 6715 | *Streptococcus sobrinus*/g | 10⁵ | + |
| 6715 | *Streptococcus sobrinus*/g | 10⁴ | - |
| Ingbritt | *Streptococcus mutans*/c | 10⁸ | +++ |
| Ingbritt | *Streptococcus mutans*/c | 10⁷ | ++ |
| Ingbritt | *Streptococcus mutans*/c | 10⁶ | + |
| P4 | *Streptococcus mutans*/e | 10⁸ | ++ |
| P4 | *Streptococcus mutans*/e | 10⁷ | + |
| P4 | *Streptococcus mutans*/e | 10⁶ | + |
| OMZ175 | *Streptococcus mutans*/f | 10⁸ | ++ |
| OMZ175 | *Streptococcus mutans*/f | 10⁷ | + |
| OMZ175 | *Streptococcus mutans*/f | 10⁶ | - |
| ATCC10556 | *Streptococcus sanguis* | 10⁹ | - |
| IFO14252 | *Streptococcus salivarius* | 10⁹ | - |
| ATCC49456 | *Streptococcus mitis* | 10⁹ | - |
| ATCC35037 | *Streptococcus oralis* | 10⁹ | - |
| Charis | *Streptococcus gordonii* | 10⁹ | - |

It can be seen from Table 10 that the reactivity of the unpurified polyclonal antibody with *Streptococcus sobrinus is* not greater than 10 times its reactivity with *Streptococcus mutans.* Moreover, it can be seen from Table 11 that, when immunochromatographic strips incorporating the unpurified polyclonal antibody were used, its reaction with *Streptococcus mutans* is detected and it is impossible to detect *Streptococcus sobrinus* specifically.

### Example 2

### Detection of Streptococcus sobrinus from dental plaque by means of immunochromatographic strips

### (1) Preparation of test fluids containing dental plaque

In 120 human subjects, the oral cavity was washed with water to remove saliva components, and dental plaque was collected with a spatula. After weighing, the dental plaque was suspended in PBS so as to give a concentration of 1 mg(wet weight)/ml, and ultrasonicated at 60 W for 20 seconds. Thus, there were obtained test fluids containing dental plaque.

### (2) Assay of Streptococcus sobrinus present in plaque sample solutions by a cultivation process

After each of the plaque-containing test fluids obtained according to the procedure described in the above step (1) was suitably diluted, 100 µl each of the diluted test fluid was spread over a Mitis-Salivarius-Bacitracin (hereinafter also referred to as "MSB") solid medium and a BHI solid medium, and cultured at 37°C for 24 hours under anaerobic conditions. After the numbers of colonies formed on the MSB and BHI solid media, the concentration (in cells/ml) of mutans streptococci was calculated on the basis of the dilution ratio of the test fluid. On the MSB and BHI media, *Streptococcus sobrinus* and *Streptococcus mutans* were distinguished according to the morphological classification of colonies. With respect to morphologically indistinguishable colonies, a pure culture of each colony was made. Thereafter, *Streptococcus sobrinus* and *Streptococcus mutans* were identified according to an immunoassay technique using antibodies specific for the serotypes of mutans streptococci and a biochemical technique such as a sugar fermentation test.

### (3) Assay of Streptococcus sobrinus present in plaque-containing test fluids by means of immunochromatographic strips

On the basis of the results of examination in the above step (2), 14 samples were selected. They include 8 samples in which *Streptococcus sobrinus* was detected, 5 samples in which *Streptococcus sobrinus* was not detected, but *Streptococcus mutans* was detected, and 1 sample in which neither of them was detected, For 200 µl each of test fluids containing these samples, *Streptococcus sobrinus* was assayed by using immunochromatographic strips prepared according to the procedure described in step (2) of Example 1. After 10 minutes, the spot color intensities produced on the immunochromatographic strips were rated in four grades (+++: strongly positive; ++: positive; +: weakly positive; -: negative) according to the criteria shown in Table 1 above. These ratings were compared with the concentrations of *Streptococcus sobrinus* as determined by the cultivation process described in step (2). The results thus obtained are shown in Table 12.

**Table 12**

| Sample | Cultivation process | | Immunochromatographic assay |
|---|---|---|---|
| | *S. sobrinus* (x10⁵ cells/ml) | *S. mutans* (x10⁵ cells/ml) | Rating |
| 1 | 138.0 | 122.0 | +++ |
| 2 | 36.8 | 16.0 | ++ |
| 3 | 19.2 | 139.0 | + |
| 4 | 3.38 | 18.0 | + |
| 5 | 2.24 | 29.0 | + |
| 6 | 2.09 | 8.0 | + |
| 7 | 1.84 | 1.52 | + |
| 8 | 1.6 | 7.0 | + |
| 9 | 0.00 | 52.8 | - |
| 10 | 0.00 | 3.92 | - |
| 11 | 0.00 | 0.88 | - |
| 12 | 0.00 | 0.78 | - |
| 13 | 0.00 | 0.01 | - |
| 14 | 0.00 | 0.00 | - |

As can be seen from Table 12, the resulting spot color intensities correlate with the concentrations of *Streptococcus sobrinus as* determined by the cultivation process, but do not correlate with the concentrations of *Streptococcus mutans*. Thus, when test fluids containing *Streptococcus sobrinus* at a concentration of not less than 10⁵ cells/ml are assayed by means of immunochromatographic strips in accordance with the present invention, *Streptococcus sobrinus* can be concentration-dependently and specifically detected.

### Preparation Example 2

### Preparation of a polyclonal antibody against Streptococcus mutans and evaluation of the reactivity of the antibody

### (1) Preparation of cell sample suspensions

A cell sample suspension of Ingbritt (*Streptococcus mutans,* serotype c) was prepared in the same manner as in step (1) of Preparation Example 1. After this cell sample suspension was adjusted to A₆₀₀=1.0, its cell concentration was found to be about 2x10⁹ cells/ml. According to substantially the same procedure, cell sample suspensions were prepared by using B13 (*Streptococcus sobrinus,* serotype d), 6715 (*Streptococcus sobrinus*, serotype g), P4 (*Streptococcus mutans*, serotype e), OMZ175 (*Streptococcus mutans,* serotype f), ATCC10556 (*Streptococcus sanguis*), IFO14252 (*Streptococcus salivarius*), ATCC49456 (*Streptococcus mitis*), ATCC35037 (*Streptococcus oralis*) and Charis (*Streptococcus gordonil*).

### (2) Preparation of an antiserum against Streptococcus mutans

A cell suspension of Ingbritt having a concentration of 10 mg(wet cells)/ml was prepared in the same manner as in step (2) of Preparation Example 1. Then, an antigenic cell suspension was formed by diluting the above cell suspension twofold with 0.5% formalin-PBS so as to give a concentration of 5 mg(wet cells)/ml. Using this antigenic cell suspension, a rabbit was immunized in the same manner as in step (2) of Preparation Example 1. Thus, there was obtained an antiserum against *Streptococcus mutans.*

### (3) Purification of a polyclonal antibody against Streptococcus mutans

Using the cell sample suspensions prepared in step (1), 20 ml of a cell suspension containing B13 (*Streptococcus sobrinus*, serotype d), 6715 (*Streptococcus sobrinus*, serotype g), ATCC10556 (*Streptococcus sanguis*), IFO14252 (*Streptococcus salivarius*), ATCC49456 (*Streptococcus mitis*), ATCC35037 (*Streptococcus oralis*) and Charis (*Streptococcus gordonii*), each at a concentration of about 2x10¹² cells/ml, was prepared. This cell suspension was mixed with 0.5 ml of the antiserum obtained in the above step (2), and the resulting mixture was reacted at 4°C for 60 minutes. After this mixture was centrifuged at 4,000g for 5 minutes, the supernatant was separated and filtered through a 0.22 µm filter.
Subsequently, the supernatant was treated with protein A in the same manner as in step (3) of Preparation Example 1. Thus, about 5 mg of IgG was recovered from 0.5 ml of the antiserum.

### (4) antibody against Streptococcus mutans by direct ELISA Evaluation of the polyclonal

The reactivity of the polyclonal antibody against *Streptococcus mutans* (hereinafter also referred to as "αIngbritt") purified in the above step (3) with various bacterial strains was evaluated according to the following direct ELISA process.
Specifically, first of all, the various cell sample suspensions obtained in the above step (1) were diluted with a 0.1M carbonate buffer solution (pH 9.0) so as to give concentrations of 1x10⁴ to 10⁹ cells/ml. 50 µl each of these diluted cell suspensions were added to wells of a 96-well immunoplate (Nunc; Maxisorp) and immobilized by allowing the immunoplate to stand at 4°C for 12 hours. Thereafter, the immunoplate was freed of the cell suspensions, and washed three times with 300 µl portions of PBS.
Then, 300 µl of a 2% BSA-0.1M carbonate buffer solution (pH 9.0) was added to each well of the immunoplate, and allowed to stand at 37°C for 2 hours. Thereafter, the immunoplate was freed of the 2% BSA-carbonate buffer solution (pH 9.0), and washed three times with 300 µl portions of 0.05% Tween 20-PBS (pH 7.4).
Then, αIngbritt was diluted with 1% BSA-0.05% Tween 20-PBS (pH 7.4) so as to give a concentration of 1.0 µg/ml. 50 µl of this solution was added to each well of the immunoplate, and allowed to stand at 37°C for 1 hour. Thereafter, the immunoplate was freed of the solution, and washed three times with 300 µl portions of 0.05% Tween 20-PBS (pH 7.4).
Then, an alkaline phosphatase-labeled anti-rabbit IgG (Fc) polyclonal antibody (goat) (Cappel) was diluted with 1% BSA-0.05% Tween 20-PBS (pH 7.4) so as to give a concentration of 10 µg/ml. 50 µl of this solution was added to each well of the immunoplate, and allowed to stand at 37°C for 1 hour. Thereafter, the immunoplate was freed of the solution, and washed three times with 300 µl portions of 0.05% Tween 20-PBS (pH 7.4).

Then, 50 µl of a color-producing substrate solution comprising an aqueous 2-ethanolamine solution of p-nitrophenyl phosphate (BIORAD) was added to each well, and reacted at room temperature for 20 minutes. After completion of the reaction, the reaction was stopped by adding 50 µl of 0.4 M NaOH to each well, and the absorbance at 405 nm was measured. The results thus obtained are shown in Table 13.

**Table 13**

| Strain | Species/serotype | Quantity (cells/well) | A₄₀₅ |
|---|---|---|---|
| Ingbritt | *Streptococcus mutans*/c | 5×10³ | 0.005 |
| Ingbritt | *Streptococcus mutans*/c | 5×10⁴ | 0.018 |
| Ingbritt | *Streptococcus mutans*/c | 5×10⁵ | 0.092 |
| Ingbritt | *Streptococcus mutans*/c | 5×10⁶ | 0.736 |
| Ingbritt | *Streptococcus mutans*/c | 5×10⁷ | 1.824 |
| P4 | *Streptococcus mutans*/e | 5×10³ | 0.004 |
| P4 | *Streptococcus mutans*/e | 5×10⁴ | 0.016 |
| P4 | *Streptococcus mutans*/e | 5×10⁵ | 0.081 |
| P4 | *Streptococcus mutans*/e | 5×10⁶ | 0.613 |
| P4 | *Streptococcus mutans*/e | 5×10⁷ | 1.475 |
| OMZ175 | *Streptococcus mutans*/f | 5×10³ | 0.004 |
| OMZ175 | *Streptococcus mutans*/f | 5×10⁴ | 0.010 |
| OMZ175 | *Streptococcus mutans*/f | 5×10⁵ | 0.065 |
| OMZ175 | *Streptococcus mutans*/f | 5×10⁶ | 0.455 |
| OMZ175 | *Streptococcus mutans*/f | 5×10⁷ | 1.103 |
| B13 | *Streptococcus sobrinus*/d | 5×10⁵ | 0.002 |
| B13 | *Streptococcus sobrmus*/d | 5×10⁶ | 0.004 |
| B13 | *Streptococcus sobrinus*/d | 5×10⁷ | 0.010 |
| 6715 | *Streptococcus sobrinus*/g | 5×10⁵ | 0.003 |
| 6715 | *Streptococcus sobrinus*/g | 5×10⁶ | 0.004 |
| 6715 | *Streptococcus sobrinus*/g | 5×10⁷ | 0.008 |
| ATCC10556 | *Streptococcus sanguis* | 5×10⁷ | 0.004 |
| IFO14252 | *Streptococcus salivarius* | 5×10⁷ | 0.003 |
| ATCC49456 | *Streptococcus mitis* | 5×10⁷ | 0.004 |
| ATCC35037 | *Streptococcus oralis* | 5×10⁷ | 0.008 |
| Charis | *Streptococcus gordonii* | 5×10⁷ | 0.010 |

It can be seen from Table 13 that, when an antigen-antibody reaction is detected by using 5x10⁵ cells/well of *Streptococcus mutans* as the antigen, a higher degree of reaction is detected than when 5x10⁷ cells/well of other oral streptococci are used as antigens. This indicates that the reactivity of αIngbritt with *Streptococcus mutans* is not less than 100 times its reactivity with other oral streptococci.

### Example 3

### Preparation and evaluation of immunochromatographic strips for the simultaneous assay of Streptococcus sobrinus and Streptococcus mutans

### (1) Preparation of a gold colloid-labeled polyclonal antibody against Streptococcus mutans

A gold colloid-labeled product of αIngbritt (hereinafter also referred to as the "gold colloid-labeled αingbritt") was prepared in the same manner as in step (1) of Example 1, and preserved at 4°C.

### (2) Preparation of an immunochromatographic strip

At a detection line 6 formed on a development membrane 4 comprising a nitricellulose membrane (MILLIPORE; High-Flow Membrane, 25 mm x 6 mm), 1 µl of α6715 and αIngbritt each having a concentration of 1 mg/ml were spotted so that they were arranged in parallel as viewed in the direction of development. In addition, 1 µl of anti-rabbit IgG(H+L) polyclonal antibody having a concentration of 1 mg/ml was spotted at a control judgment line 7 formed on development membrane 4. Then, the membrane was dried in an incubator at 37°C for 60 minutes to immobilize the antibodies thereto. This antibody-immobilized membrane was shaken in an aqueous 1% skim milk-0.1% Triton X100 solution at room temperature for 5 minutes. Then, the membrane was shaken in a 10 mM phosphate buffer solution (pH 7.4) at room temperature for 10 minutes. Thereafter, the membrane was taken out and dried in a dessicator for 60 minutes under aspiration with a vacuum pump.
Moreover, a conjugate pad 3 (MILLIPORE; 7.5 mm x 6 mm) was shaken in an aqueous 0.5% PVA-0.5% sucrose solution for 1 minute. Thereafter, the conjugate pad was taken out and dried in a dessicator for 60 minutes under aspiration with a vacuum pump. After 12.5 µl of gold colloid-labeled α6715 adjusted to A₅₂₀=2.0 and 12.5 µl of gold colloid-labeled αIngbritt adjusted to A₅₂₀=2.0 were added to the conjugate pad, it was dried in a dessicator for 60 minutes under aspiration with a vacuum pump. Furthermore, a sample pad 2 (MILLIPORE; 17 mm x 6 mm) was shaken in an aqueous 1% Tween 20-PBS solution for 1 minute. Thereafter, the sample pad was taken out and dried in a dessicator for 60 minutes under aspiration with a vacuum pump. An absorption pad 5 (MILLIPORE; 20 mm x 6 mm) was used in its as-received condition.
The immunochromatographic strip components as shown in FIG. 4, which were prepared in the above-described manner, were placed on a plastic support and assembled into an immunochromatographic strip as shown in FIG. 2.

### (3) Evaluation of the specificity and sensitivity of immunochromatographic strips

A plurality of immunochromatographic strips were prepared in the above-described manner. Cells of various reference strains were suspended in PBS, and 100 µl of each suspension was added to sample pad 2 of each immunochromatographic strip. After 10 minutes, the presence or absence of a spot was judged. Specifically, The sensitivity and quantifying ability of the immunochromatographic strip were evaluated by examining the amounts of gold colloid captured on the immobilized antibody spots by visual observation and rating them in four grades (+++: strongly positive; ++: positive; +: weakly positive; -: negative). The results thus obtained are shown in Table 14.

**Table 14**

| Strain | Species/serotype | Cell concentration of test fluid (cells/ml) | Rating for detection line 6a (detection of *S. sobrinus*) | Rating for detection line 6b (detection of *S. mutans*) |
|---|---|---|---|---|
| B13 | *Streptococcus sobrinus*/d | 10⁹ | +++ | - |
| B13 | *Streptococcus sobrinus*/d | 10⁸ | +++ | - |
| B13 | *Streptococcus sobrinus*/d | 10⁷ | +++ | - |
| B13 | *Streptococcus sobrinus*/d | 10⁶ | ++ | - |
| B13 | *Streptococcus sobrinus*/d | 10⁵ | + | - |
| B13 | *Streptococcus sobrinus*/d | 10⁴ | - | - |
| 6715 | *Streptococcus sobrinus*/g | 10⁹ | +++ | - |
| 6715 | *Streptococcus sobrinus*/g | 10⁸ | +++ | - |
| 6715 | *Streptococcus sobrinus*/g | 10⁷ | +++ | - |
| 6715 | *Streptococcus sobrinus*/g | 10⁶ | ++ | - |
| 6715 | *Streptococcus sobrinus*/g | 10⁵ | + | - |
| 6715 | *Streptococcus sobrinus*/g | 10⁴ | - | - |
| Ingbritt | *Streptococcus mutans*/c | 10⁹ | - | +++ |
| Ingbritt | *Streptococcus mutans*/c | 10⁸ | - | ++ |
| Ingbritt | *Streptococcus mutans*/c | 10⁷ | - | + |
| Ingbritt | *Streptococcus mutans*/c | 10⁶ | - | + |
| Ingbritt | *Streptococcus mutans*/c | 10⁵ | - | - |
| P4 | *Streptococcus mutans*/e | 10⁹ | - | +++ |
| P4 | *Streptococcus mutans*/e | 10⁸ | - | ++ |
| P4 | *Streptococcus mutans*/e | 10⁷ | - | + |
| P4 | *Streptococcus mutans*/e | 10⁶ | - | + |
| P4 | *Streptococcus mutans*/e | 10⁵ | - | - |
| OMZ175 | *Streptococcus mutans*/f | 10⁹ | - | +++ |
| OMZ175 | *Streptococcus mutans*/f | 10⁸ | - | ++ |
| OMZ175 | *Streptococcus mutans*/f | 10⁷ | - | + |
| OMZ175 | *Streptococcus mutans*/f | 10⁶ | - | - |
| OMZ175 | *Streptococcus mutans*/f | 10⁵ | - | - |
| ATCC10556 | *Streptococcus sanguis* | 10⁹ | - | - |
| IF014252 | *Streptococcus salivarius* | 10⁹ | - | - |
| ATCC49456 | *Streptococcus mitis* | 10⁹ | - | - |
| ATCC35037 | *Streptococcus oralis* | 10⁹ | - | - |
| Charis | *Streptococcus gordonii* | 10⁹ | - | - |

It can be seen from Table 14 that, when *Streptococcus sobrinus* and *Streptococcus mutans* present in test fluids are detected, *Streptococcus sobrinus* present in test fluids having a cell concentration in the range of 1x10⁵ cells/ml to 1x10⁹ cells/ml is specifically detected on the *Streptococcus sobrinus* detection spot formed at detection line 6. Moreover, it can also be seen that, on the *Streptococcus mutans* detection spot, *Streptococcus mutans* present in test fluids is specifically detected in the concentration range of 1x10⁶ cells/ml to 1x10⁹ cells/ml for Ingbritt (serotype c) and P4 (serotype e) and in the concentration range of 1x10⁷ cells/ml to 1x10⁹ cells/ml for OMZ175 (serotype f). In order to enhance the detection sensitivity for *Streptococcus mutans* to the same level as the detection sensitivity for *Streptococcus sobrinus* (i.e., 10⁵ cells/well), it is necessary to prepare an antibody having higher reactivity. The reactivity of this antibody must be such that, when 5x10³ cells/ml of *Streptococcus mutans is* assayed by direct ELISA as described in step (4) of Preparation Example 2, a reaction above background level is detected.

### Example 4

### Simultaneous determination of the quantities of Streptococcus sobrinus and Streptococcus mutans present in plaque-containing test fluids by means of immunochromatographic strips

The same 14 samples as used in Example 2 were assayed. For 200 µl each of test fluids containing these samples, the quantity of *Streptococcus sobrinus* and the quantity of *Streptococcus mutans* were simultaneously determined by using immunochromatographic strips prepared according to the procedure described in step (2) of Example 3. After 10 minutes, the spot color intensities produced on the immunochromatographic strips were rated in four grades (+++: strongly positive; ++: positive; +: weakly positive; -: negative). These ratings were compared with the concentrations of *Streptococcus sobrinus* and *Streptococcus mutans* as determined by the cultivation process described previously. The results thus obtained are shown in Table 15.

**Table 15**

| Sample | Cultivation process | | Immunochromatographic assay | |
|---|---|---|---|---|
| | *S. sobrinus* (x10⁵ cells/ml) | *S. mutans* (x10⁵ cells/ml) | Rating (detection line 6a), detection of *S. sobrinus* | Rating (detection line 6b), detection of *S. mutans* |
| 1 | 138.0 | 122.0 | +++ | ++ |
| 2 | 36.8 | 16.0 | ++ | + |
| 3 | 19.2 | 139.0 | ++ | ++ |
| 4 | 3.38 | 18.0 | + | + |
| 5 | 2.24 | 29.0 | + | + |
| 6 | 2.09 | 8.0 | + | + |
| 7 | 1.84 | 1.52 | + | - |
| 8 | 1.6 | 7.0 | + | + |
| 9 | 0.00 | 52.8 | - | ++ |
| 10 | 0.00 | 3.92 | - | - |
| 11 | 0.00 | 0.88 | - | - |
| 12 | 0.00 | 0.78 | - | - |
| 13 | 0.00 | 0.01 | - | - |
| 14 | 0.00 | 0.00 | - | - |

As can be seen from Table 15, on the *Streptococcus sobrinus* detection spot, the resulting spot color intensities correlate with the concentrations of *Streptococcus sobrinus*, regardless of the concentrations of *Streptococcus mutans* as determined by the cultivation process. Moreover, on the *Streptococcus mutans* detection spot, not less than 7x10⁵ cells/ml of *Streptococcus mutans* is detected, regardless of the concentrations of *Streptococcus sobrinus* as determined by the cultivation process. Thus, when test fluids containing *Streptococcus sobrinus* and *Streptococcus mutans* at a concentration of not less than 10⁵ cells/ml are assayed by means of immunochromatographic strips for simultaneous assay purposes in accordance with the present invention, not less than 10⁵ cells/ml of *Streptococcus sobrinus* can be concentration-dependently and specifically detected, and not less than 7x10⁵ cells/ml of *Streptococcus mutans* can be simultaneously and specifically detected.

### Preparation Example 3

### Preparation of a polyclonal antibody reacting with Streptococcus mutans and Streptococcus sobrinus and evaluation of the reactivity of the antibody

A polyclonal antibody reacting with *Streptococcus mutans* and *Streptococcus sobrinus* (hereinafter also referred to as "αIngbritt-6715") was prepared by mixing 10 mg of αIngbritt prepared in Preparation Example 2 with 1 mg of α6715 prepared in Preparation Example 1. The reactivity of αIngbritt-6715 with various bacterial strains was evaluated according to the same direct ELISA process as described in step (4) of Preparation Example 2. The results thus obtained are shown in Table 16.

**Table 16**

| Strain | Species/serotype | Quantity (cells/well) | A₄₀₅ |
|---|---|---|---|
| Ingbritt | *Streptococcus mutans*/c | 5×10³ | 0.005 |
| Ingbritt | *Streptococcus mutans*/c | 5×10⁴ | 0.013 |
| Ingbritt | *Streptococcus mutans*/c | 5×10⁵ | 0.070 |
| Ingbritt | *Streptococcus mutans*/c | 5×10⁶ | 0.680 |
| Ingbritt | *Streptococcus mutans*/c | 5×10⁷ | 1.535 |
| P4 | *Streptococcus mutans*/e | 5×10³ | 0.005 |
| P4 | *Streptococcus mutans*/e | 5×10⁴ | 0.012 |
| P4 | *Streptococcus mutans*/e | 5×10⁵ | 0.065 |
| P4 | *Streptococcus mutans*/e | 5×10⁶ | 0.624 |
| P4 | *Streptococcus mutans*/e | 5×10⁷ | 1.324 |
| OMZ175 | *Streptococcus mutans*/f | 5×10³ | 0.004 |
| OMZ175 | *Streptococcus mutans*/f | 5×10⁴ | 0.010 |
| OMZ175 | *Streptococcus mutans*/f | 5×10⁵ | 0.048 |
| OMZ175 | *Streptococcus mutans*/f | 5×10⁶ | 0.478 |
| OMZ175 | *Streptococcus mutans*/f | 5×10⁷ | 1.085 |
| B13 | *Streptococcus sobrinus*/d | 5×10³ | 0.005 |
| B13 | *Streptococcus sobrinus*/d | 5×10⁴ | 0.014 |
| B13 | *Streptococcus sobrinus*/d | 5×10⁵ | 0.068 |
| B13 | *Streptococcus sobrinus*/d | 5×10⁶ | 0.625 |
| B13 | *Streptococcus sobrinus*/d | 5×10⁷ | 1.398 |
| 6715 | *Streptococcus sobrinus*/g | 5×10³ | 0.005 |
| 6715 | *Streptococcus sobrinus*/g | 5×10⁴ | 0.013 |
| 6715 | *Streptococcus sobrinus*/g | 5×10⁵ | 0.071 |
| 6715 | *Streptococcus sobrinus*/g | 5×10⁶ | 0.641 |
| 6715 | *Streptococcus sobrinus*/g | 5×10⁷ | 1.435 |
| ATCC10556 | *Streptococcus sanguis* | 5×10⁷ | 0.004 |
| IFO14252 | *Streptococcus salivarius* | 5×10⁷ | 0.003 |
| ATCC49456 | *Streptococcus mitis* | 5×10⁷ | 0.004 |
| ATCC35037 | *Streptococcus oralis* | 5×10⁷ | 0.006 |
| Charis | *Streptococcus gordonii* | 5×10⁷ | 0.007 |

It can be seen from Table 16 that, when an antigen-antibody reaction is detected by using 5x10⁵ cells/well of *Streptococcus mutans* or *Streptococcus sobrinus* as the antigen, a higher degree of reaction is detected than when 5x10⁷ cells/well of other oral streptococci are used as antigens. This indicates that the reactivity of αIngbritt-6715 with *Streptococcus mutans* or *Streptococcus sobrinus* is not less than 100 times its reactivity with other oral streptococci. Moreover, when antigen-antibody reactions are detected by using an equal quantity, in the range of 5x10⁵ cells/well to 5x10⁷ cells/well, of *Streptococcus mutans* and *Streptococcus sobrinus* as the antigens, the mutual ratio between the degrees of reaction detected from *Streptococcus mutans* and *Streptococcus sobrinus* is within 1.5. This indicates that the reactivity of αIngbritt-6715 with *Streptococcus mutans* is almost equal to its reactivity with *Streptococcus sobrinus*.

### Example 5

### Preparation and evaluation of immunochromatographic strips for the simultaneous determination of the quantity of Streptococcus sobrinus and the total quantity of Streptococcus mutans and Streptococcus sobrinus

### (1) Preparation of a gold colloid-labeled polyclonal antibody reacting with Streptococcus mutans and Streptococcus sobrinus

A gold colloid-labeled product of αIngbritt-6715 (hereinafter also referred to as the "gold colloid-labeled αingbritt-6715") was prepared in the same manner as in step (1) of Example 1, and preserved at 4°C.

### (2) Preparation of an immunochromatographic strip

At a detection line 6 formed on a development membrane 4 comprising a nitricellulose membrane (MILLIPORE; High-Flow Membrane, 25 mm x 6 mm), 1 µl of α6715 and αIngbritt-6715 each having a concentration of 1 mg/ml were spotted so that they were arranged in parallel as viewed in the direction of development. In addition, 1 µl of anti-rabbit IgG(H+L) polyclonal antibody having a concentration of 1 mg/ml was spotted at a control judgment line 7 formed on development membrane 4. Moreover, 12.5 µl of gold colloid-labeled α6715 adjusted to A₅₂₀=2.0 and 12.5 µl of gold colloid-labeled αIngbritt-6715 adjusted to A₅₂₀=2.0 were added to a conjugate pad 3. Except for these operations, the procedure described in step (2) of Example 1 was repeated to prepare immunochromatographic strip components as shown in FIG. 4. These components were placed on a plastic support and assembled into an immunochromatographic strip as shown in FIG. 2.

### (3) Evaluation of the specificity and sensitivity of immunochromatographic strips

A plurality of immunochromatographic strips were prepared in the above-described manner. Cells of various reference strains were suspended in PBS, and 100 µl of each suspension was added to sample pad 2 of each immunochromatographic strip. After 10 minutes, the presence or absence of a spot was judged. Specifically, The sensitivity and quantifying ability of the immunochromatographic strip were evaluated by examining the amounts of gold colloid captured on the immobilized antibody spots by visual observation and rating them in four grades (+++: strongly positive; ++: positive; +: weakly positive; -: negative). The results thus obtained are shown in Table 17.

**Table 17**

| Strain | Species/serotype | Cell concentration of test fluid (cells/ml) | Rating for detection line 6a (detection of *S. sobrinus*) | Rating for detection line 6b (detection of total amount of *S. mutans* and *S. sobrinus*) |
|---|---|---|---|---|
| B13 | *Streptococcus sobrinus*/d | 10⁹ | +++ | +++ |
| B13 | *Streptococcus sobrinus*/d | 10⁸ | +++ | ++ |
| B13 | *Streptococcus sobrinus*/d | 10⁷ | +++ | + |
| B13 | *Streptococcus sobrinus*/d | 10⁶ | ++ | + |
| B13 | *Streptococcus sobrinus*/d | 10⁵ | + | - |
| B13 | *Streptococcus sobrinus*/d | 10⁴ | - | - |
| 6715 | *Streptococcus sobrinus*/g | 10⁹ | +++ | +++ |
| 6715 | *Streptococcus sobrinus*/g | 10⁸ | +++ | ++ |
| 6715 | *Streptococcus sobrinus*/g | 10⁷ | +++ | + |
| 6715 | *Streptococcus sobrinus*/g | 10⁶ | ++ | + |
| 6715 | *Streptococcus sobrinus*/g | 10⁵ | + | - |
| 6715 | *Streptococcus sobrinus*/g | 10⁴ | - | - |
| Ingbritt | *Streptococcus mutans*/c | 10⁹ | - | +++ |
| Ingbritt | *Streptococcus mutans*/c | 10⁸ | - | ++ |
| Ingbritt | *Streptococcus mutans*/c | 10⁷ | - | + |
| Ingbritt | *Streptococcus mutans*/c | 10⁶ | - | + |
| Ingbritt | *Streptococcus mutans*/c | 10⁵ | - | - |
| P4 | *Streptococcus mutans*/e | 10⁹ | - | +++ |
| P4 | *Streptococcus mutans*/e | 10⁸ | - | ++ |
| P4 | *Streptococcus mutans*/e | 10⁷ | - | + |
| P4 | *Streptococcus mutans*/e | 10⁶ | - | + |
| P4 | *Streptococcus mutans*/e | 10⁵ | - | - |
| OMZ175 | *Streptococcus mutans*/f | 10⁹ | - | +++ |
| OMZ175 | *Streptococcus mutans*/f | 10⁸ | - | ++ |
| OMZ175 | *Streptococcus mutans*/f | 10⁷ | - | + |
| OMZ175 | *Streptococcus mutans*/f | 10⁶ | - | - |
| OMZ175 | *Streptococcus mutans*/f | 10⁵ | - | - |
| ATCC10556 | *Streptococcus sanguis* | 10⁹ | - | - |
| IF014252 | *Streptococcus salivarius* | 10⁹ | - | - |
| ATCC49456 | *Streptococcus mitis* | 10⁹ | - | - |
| ATCC35037 | *Streptococcus oralis* | 10⁹ | - | - |
| Charis | *Streptococcus gordonii* | 10⁹ | - | - |

It can be seen from Table 17 that, when *Streptococcus sobrinus* present in test fluids and the combination of *Streptococcus mutans* and *Streptococcus sobrinus* present therein are detected, *Streptococcus sobrinus* present in test fluids having a cell concentration in the range of 1x10⁵ cells/ml to 1x10⁹ cells/ml is specifically detected on the *Streptococcus sobrinus* detection spot formed at detection line 6. Moreover, on the *Streptococcus mutans*/*Streptococcus sobrinus* detection spot, a positive reaction is observed for test fluids containing *Streptococcus mutans* or *Streptococcus sobrinus* at a concentration of 1x10⁷ cells/ml or in the range of 1x10⁶ cells/ml to 1x10⁹ cells/ml. Since test fluids containing other oral streptococci at a concentration of 1x10⁹ cells/ml exhibit a negative reaction, this indicates that *Streptococcus mutans* and *Streptococcus sobrinus* present in test fluids can be specifically detected.

### Example 6

### Simultaneous determination of the quantity of Streptococcus sobrinus present in plaque-containing test fluids and the total quantity of Streptococcus mutans and Streptococcus sobrinus present therein by means of immunochromatographic strips

The same 14 samples as used in Example 2 were assayed. For 200 µl each of test fluids containing these samples, the quantity of *Streptococcus sobrinus* and the total quantity of *Streptococcus mutans* and *Streptococcus sobrinus* were simultaneously determined by using immunochromatographic strips prepared according to the procedure described in step (2) of Example 5. After 10 minutes, the spot color intensities produced on the immunochromatographic strips were rated in four grades (+++: strongly positive; ++: positive; +: weakly positive; -: negative). These ratings were compared with the concentrations of *Streptococcus sobrinus* and *Streptococcus mutans* as determined by the cultivation process described previously. The results thus obtained are shown in Table 18.

**Table 18**

| Sample | Cultivation process | | | Immunochromatographic assay | |
|---|---|---|---|---|---|
| | *S. sobrinus* (x10⁵ cells/ml) | *S. mutans* (x10⁵ cells/ml) | Total quantity of *mutans* and *sobrinus* (x10⁵ cells/ml) | Rating for detection line 6a (detection of *S. sobrinus*) | Rating for detection line 6b (detection of total amount of *S. mutans* / *S. sobrinus*) |
| 1 | 138.0 | 122.0 | 260.0 | +++ | +++ |
| 2 | 36.8 | 16.0 | 52.8 | ++ | + |
| 3 | 19.2 | 139.0 | 158.2 | ++ | ++ |
| 4 | 3.38 | 18.0 | 21.38 | + | + |
| 5 | 2.24 | 29.0 | 31.24 | + | + |
| 6 | 2.09 | 8.0 | 10.09 | + | + |
| 7 | 1.84 | 1.52 | 3.36 | + | - |
| 8 | 1.6 | 7.0 | 8.6 | + | - |
| 9 | 0.00 | 52.8 | 52.8 | - | + |
| 10 | 0.00 | 3.92 | 3.92 | - | - |
| 11 | 0.00 | 0.88 | 0.88 | - | - |
| 12 | 0.00 | 0.78 | 0.78 | - | - |
| 13 | 0.00 | 0.01 | 0.01 | - | - |
| 14 | 0.00 | 0.00 | 0.00 | - | - |

As can be seen from Table 18, on the *Streptococcus sobrinus* detection spot, the resulting spot color intensities correlate with the concentrations of *Streptococcus sobrinus*, regardless of the concentrations of *Streptococcus mutans* as determined by the cultivation process. Moreover, on the *Streptococcus mutans*/*Streptococcus sobrinus* detection spot, a specific reaction is detected when the total quantity is not less than 10⁶ cells/ml. Thus, when test fluids having a *Streptococcus sobrinus* concentration of not less than 10⁵ cells/ml and a *Streptococcus mutans*/*Streptococcus sobrinus* total concentration of not less than 10⁵ cells/ml are assayed by means of immunochromatographic strips for simultaneous assay purposes in accordance with the present invention, not less than 10⁵ cells/ml of *Streptococcus sobrinus* can be concentration-dependently and specifically detected, and a specific reaction can be detected when the total quantity of *Streptococcus mutans* and *Streptococcus sobrinus* is not less than 10⁶ cells/ml.

### Industreal Applicability

According to the present invention, *Streptococcus sobrinus* present in test fluids containing saliva or dental plaque in which *Streptococcus mutans* coexists can be directly assayed with high sensitivity and without resorting to a cultivation process or the like. In particular, the assay methods of the present invention which are based on immunochromatographic assay, latex agglutination assay and flow-through immunoassay make it possible to assay *Streptococcus sobrinus* rapidly and simply in dental clinics and at home. Moreover, the use of quantitative latex assay and ELISA permits a large number of test fluids to be rapidly assayed for *Streptococcus sobrinus*. Furthermore, according to the present invention, the quantity of *Streptococcus sobrinus* present in test fluids and the quantity of *Streptococcus mutans* present therein (or the total quantity of *Streptococcus mutans* and *Streptococcus sobrinus* present therein) can be determined at the same time. Thus, the present invention makes it possible to construct a new diagnostic system for judging the degree of risk of dental caries.
Accordingly, the present invention can be utilized in the field of medical diagnostics, as well as in industries concerned with the manufacture of reagents and devices used in this medical field.

## Claims

1. A method for the detection of *Streptococcus sobrinus* in a test fluid suspected of containing *Streptococcus sobrinus* and *Streptococcus mutans,* said method comprising the steps of
(A) providing an antibody whose binding ability for *Streptococcus sobrinus* is not less than 100 times that for *Streptococcus mutans;*
(B) bringing the antibody into contact with the test fluid to form an immune complex; and
(C) assaying the immune complex.

2. A method as claimed in claim 1 wherein the antibody whose binding ability for *Streptococcus sobrinus* is not less than 100 times that for *Streptococcus mutans* is a polyclonal antibody.

3. A method as claimed in claim 2 wherein the binding ability for *Streptococcus sobrinus* is determined with respect to the serotype d and g strains of the bacterial species, and the mutual ratio between the binding abilities for the serotype d and g strains is within 2.

4. A method as claimed in claim 1 wherein the test fluid suspected of containing *Streptococcus sobrinus* and *Streptococcus mutans* contains *Streptococcus sobrinus* at a concentration of 10⁵ to 10⁷ cells/ml.

5. A method as claimed in any one of claims 1 to 4 wherein the immune complex is assayed by an immunochromatographic technique.

6. A diagnostic method for judging the degree of risk of dental caries in a human subject, said method comprising the steps of
(a) preparing a test fluid derived from the saliva and/or dental plaque of the subject;
(b) providing an antibody whose binding ability for *Streptococcus sobrinus* is not less than 100 times that for *Streptococcus mutans*;
(c) bringing the test fluid prepared in step (a) into contact with the antibody provided in step (b) to form an immune complex; and
(d) assaying the immune complex, and evaluating its amount as an index to a risk of dental caries.

7. A diagnostic method as claimed in claim 6 wherein the antibody whose binding ability for *Streptococcus sobrinus* is not less than 100 times that for *Streptococcus mutans* is a polyclonal antibody.

8. A diagnostic method as claimed in claim 7 wherein the binding ability for *Streptococcus sobrinus* is determined with respect to the serotype d and g strains of the bacterial species, and the mutual ratio between the binding abilities for the serotype d and g strains is within 2.

9. A diagnostic method as claimed in claim 6 wherein the test fluid contains *Streptococcus sobrinus* at a concentration of 10⁵ to 10⁷ cells/ml.

10. A diagnostic method as claimed in any one of claims 6 to 9 wherein step (c) is carried out in the coexistence of the antibody (S antibody) with an antibody binding specifically with *Streptococcus mutans* (M antibody), or in addition to step (c), another step similar to step (c) is carried out by using M antibody in place of S antibody; the resulting immune complex derived from M antibody is also assayed; and the amount of this complex is also evaluated as an index to a risk of dental caries.

11. A diagnostic method as claimed in claim 10 wherein an antibody binding specifically with *Streptococcus mutans* and *Streptococcus sobrinus* (MS antibody) is used in place of M antibody.

12. A diagnostic method as claimed in any one of claims 6 to 11 wherein the one or more immune complexes are assayed by an immunochromatographic technique.

13. A diagnostic method as claimed in any one of claims 6 to 12 wherein the test fluid is untreated saliva.

14. An immunoassay kit or a diagnostic kit for judging the degree of risk of dental caries in human subjects, said kit including an antibody whose binding ability for *Streptococcus sobrinus is* not less than 100 times that for *Streptococcus mutans*; and if necessary, an antibody binding specifically with *Streptococcus mutans,* or an antibody binding specifically with *Streptococcus mutans* and *Streptococcus sobrinus.*

15. An immunochromatographic strip comprising a sample pad for absorbing and holding a test fluid temporarily, a conjugate pad for holding a labeled antibody temporarily, and a development membrane having a detection antibody immobilized thereto and allowing the development of the test fluid absorbed and held temporarily in the sample pad and the labeled antibody flowing out of the conjugate pad together with the test fluid, wherein the sample pad, the conjugate pad and the development membrane are joined together in the order mentioned, said immunochromatographic strip being **characterized in that** an antibody whose binding ability for *Streptococcus sobrinus* is not less than 100 times that for *Streptococcus mutans* is used as the detection antibody.

16. An immunochromatographic strip as claimed in claim 15 wherein an antibody binding specifically with *Streptococcus mutans* or an antibody binding specifically with *Streptococcus mutans* and *Streptococcus sobrinus* is concurrently used as an additional detection antibody immobilized to the development membrane

17. A polyclonal antibody whose binding ability for *Streptococcus sobrinus* is not less than 100 times that for *Streptococcus mutans.*

18. A polyclonal antibody as claimed in claim 17 wherein the binding ability for *Streptococcus sobrinus* is determined with respect to the serotype d and g strains of the bacterial species, and the mutual ratio between the binding abilities for the serotype d and g strains is within 2.
